# EUROPEAN PATENT APPLICATION

(11) **EP 0 872 485 A2**
(43) Date of publication of application: **21.10.1998**
(21) Application number: 98302866.3
(22) Date of filing: 14.04.1998
(51) Int. Cl.: C07K 14/37, C07K 14/375, C07K 14/38, C07K 14/385, C07K 14/39, C12N 15/04, C12N 15/63, C12N 1/11, C12N 1/16, C12N 1/20, C12N 5/10

(54) **IPC synthase genes from fungi**

(30) Priority: 15.04.1997 US 43591 P; 22.04.1997 US 44095 P; 13.05.1997 US 46348 P; 21.07.1997 US 53320 P; 17.10.1997 US 62971 P
(71) Applicant: ELI LILLY AND COMPANY, Indianapolis, Indiana 46285 (US)
(72) Inventor: Heider, Steven Alan, Fishers, Indiana 46038 (US); Radding, Jeffrey Alan, Carmel, Indiana 46033 (US)
(74) Representative: Denholm, Anna Marie

(57) **Abstract**

The invention provides isolated nucleic acid compounds encoding IPC synthase or subunit thereof from fungal cells. Also provided are vectors and transformed heterologous host cells for expressing IPC synthase and a method for identifying compounds that inhibit a fungal IPC synthase.

## Description

This invention relates to recombinant DNA technology. In particular the invention pertains to the isolation of novel genes and proteins that encode IPC synthase or subunit thereof from a variety of fungi and the use of said proteins in screens for inhibitors of IPC Synthase.

The incidence of life-threatening fungal infections is increasing at an alarming rate. With the exception of *Staphylococci* infections, the fungus *C. albicans* represents the fastest growing area of concern in hospital acquired bloodstream infections. About 90% of nosocomial fungal infections are caused by species of *Candida* with the remaining 10% being attributable to infections by *Aspergillus, Cryptococcus,* and *Pneumocystis.* While effective antifungal compounds have been developed for *Candida* there is growing concern that the rise in the incidence of fungal infections may portend greater resistance and virulence in the future. Moreover, anti-*Candida* compounds frequently do not possess clinically significant activity against other fungal species.

Inositolphosphoryl ceramides are sphingolipids found in a number of fungi including but not limited to S. *cerevisiae, S. pombe, C. albicans, A. fumigatus, A. nidulans* and *H. capsulatum.* A step of sphingolipid biosynthesis unique to fungi and plants is catalyzed by the enzyme IPC synthase. The IPC synthase step, covalently links inositol phosphate and ceramide, and is essential for viability in S. *cerevisiae.* Although some elements of sphingolipid biosynthesis in fungi are shared with mammalian systems, the pathways diverge at the step after formation of ceramide. Thus, the formation of inositolphosphoryl ceramide is unique to fungi and plants, making IPC synthase a good molecular target for antifungal chemotherapy.

While compounds that target IPC synthase bode well for the future of anti-fungal therapy, presently there are no clinically useful compounds that act at this step. Thus, there is a need for new compounds that inhibit IPC synthase.

The present invention relates to fungal IPC synthase and to screens for inhibitors thereof.

In one embodiment the invention relates to fungal genes that encode IPC synthase, or subunit thereof.

In another embodiment, the invention relates to fungal IPC synthase genes identified herein as SEQ ID NO:1, SEQ ID NO:4, SEQ ID NO:7, SEQ ID NO:10, SEQ ID NO:19, and SEQ ID NO:20.

In another embodiment, the invention relates to nucleic acids that are at least 70% homologous, and/or, will hybridize under high stringency conditions to a sequence identified herein as SEQ ID NO:1, SEQ ID NO:4, SEQ ID NO:7, SEQ ID NO:10, SEQ ID NO:19, and SEQ ID NO:20.

In another embodiment the present invention pertains to the proteins produced by IPC synthase genes.

In yet another embodiment, the invention relates to proteins designated herein as SEQ ID NO 2, SEQ ID NO 5, SEQ ID NO 8, SEQ ID NO 11, and SEQ ID NO:21.

In still another embodiment the invention relates to the use of purified fungal IPC synthase or subunit thereof in high throughput screens for inhibitors of fungal IPC synthase, said IPC synthase being designated herein as SEQ ID NO:2, SEQ ID NO:5, SEQ ID NO:8, SEQ ID NO:11, SEQ ID NO:14, SEQ ID NO:17, and SEQ ID NO:21.

In another embodiment the invention relates to the use of recombinant host cells that carry a vector that expresses a fungal IPC synthase in high throughput screens for inhibitors of fungal IPC synthase.

In another embodiment the invention relates to the use of the IPC synthase genes disclosed herein, or fragments thereof, as hybridization probes or PCR primers to identify and isolate homologous genes that are related in sequence and/or function.

The terms "cleavage" or "restriction" of DNA refers to the catalytic cleavage of the DNA with a restriction enzyme that acts only at certain sequences in the DNA (viz. sequence-specific endonucleases). The various restriction enzymes used herein are commercially available and their reaction conditions, cofactors, and other requirements are used in the manner well known to one of ordinary skill in the art. Appropriate buffers and substrate amounts for particular restriction enzymes are specified by the manufacturer or can readily be found in the literature.

The term "fusion protein" denotes a hybrid protein molecule not found in nature comprising a translational fusion or enzymatic fusion in which two or more different proteins or fragments thereof are covalently linked on a single polypeptide chain.

The term "plasmid" refers to an extrachromosomal genetic element. The starting plasmids herein are either commercially available, publicly available on an unrestricted basis, or can be constructed from available plasmids in accordance with published procedures. In addition, equivalent plasmids to those described are known in the art and will be apparent to the ordinarily skilled artisan.

"Recombinant DNA cloning vector" as used herein refers to any autonomously replicating agent, including, but not limited to, plasmids and phages, comprising a DNA molecule to which one or more additional DNA segments can or have been added.

The term "recombinant DNA expression vector" as used herein refers to any recombinant DNA cloning vector, for example a plasmid or phage, in which a promoter and other regulatory elements are present to enable transcription of the inserted DNA.

The term "vector" as used herein refers to a nucleic acid compound used for introducing exogenous DNA into host cells. A vector comprises a nucleotide sequence which may encode one or more protein molecules. Plasmids, cosmids, viruses, and bacteriophages, in the natural state or which have undergone recombinant engineering, are examples of commonly used vectors.

The terms "complementary" or "complementarity" as used herein refers to the capacity of purine and pyrimidine nucleotides to associate through hydrogen bonding in double stranded nucleic acid molecules. The following base pairs are complementary: guanine and cytosine; adenine and thymine; and adenine and uracil.

"Isolated nucleic acid compound" refers to any RNA or DNA sequence, however constructed or synthesized, which is locationally distinct from its natural location.

A "primer" is a nucleic acid fragment which functions as an initiating substrate for enzymatic or synthetic elongation of, for example, a nucleic acid molecule.

The term "promoter" refers to a DNA sequence which directs transcription of DNA to RNA.

A "probe" as used herein is a nucleic acid compound that hybridizes with another nucleic acid compound, and is useful for blot hybridizations, for example. A probe is at least 15 base pairs in length, its sequence being at least 90% identical with the nucleic acid molecules disclosed herein, or fragments thereof, or the complements thereof. A probe may or may not be labeled with a detectable moiety. As used herein, a probe is useful for hybridization analysis to identify sequences homologous to those disclosed herein.

The term "hybridization" as used herein refers to a process in which a single-stranded nucleic acid molecule joins with a complementary strand through nucleotide base pairing. "Selective hybridization" refers to hybridization under conditions of high stringency. The degree of hybridization depends upon, for example, the degree of complementarity, the stringency of hybridization, and the length of hybridizing strands.

The term "stringency" refers to hybridization conditions. High stringency conditions disfavor non-homologous basepairing. Low stringency conditions have the opposite effect. Stringency may be altered, for example, by temperature and salt concentration.

"Low stringency" conditions comprise, for example, a temperature of about 37° C or less, a formamide concentration of less than about 50%, and a moderate to low salt (SSC) concentration; or, alternatively, a temperature of about 50° C or less, and a moderate to high salt (SSPE) concentration.

"High stringency" conditions comprise a temperature of about 42° C or less, a formamide concentration of less than about 20%, and a low salt (SSC) concentration; or, alternatively, a temperature of about 65° C, or less, and a low salt (SSPE) concentration.

"SSC" comprises a hybridization and wash solution. 20X SSC contains 3M sodium chloride, 0.3M sodium citrate, pH 7.0.

"SSPE" comprises a hybridization and wash solution. 1X SSPE contains 180 mM NaCl, 9mM Na₂HPO₄, 0.9 mM NaH₂PO₄ and 1 mM EDTA, pH 7.4.

In yeast and other fungi, for example *C. albicans, Aspergillus nidulans, Aspergillus fumigatus, Cryptococcus neofomans, C. Krusei, C. parapsilosis, C. tropicalis,* and *C. glabrata,* IPC synthase catalyzes a step in the synthesis of inositolphosphoryl ceramide from ceramide and phosphatidylinositol (G. Becker and R. Lester, Biosynthesis of phosphoinositol-containing sphingolipid from phosphatidylinositol by a membrane prepartation from *Saccharomyces cervisiae. J. Bacteriol.* 142, 747-754, 1980). Sphingolipids are necessary for growth and viability of the yeast *S. cerevisiae.* Since IPC synthase is unique to fungi and plants it is a good target for antifungal therapy in mammals.

The IPC synthase gene of *C. glabrata* comprises the DNA sequence designated herein as SEQ ID NO 1. The IPC synthase gene of *C. Krusei* comprises the DNA sequence designated herein as SEQ ID NO 4. The IPC synthase gene of C. *parapsilosis* comprises the DNA sequence designated herein as SEQ ID NO 7. The IPC synthase gene of *C. tropicalis* comprises the DNA sequence designated herein as SEQ ID NO 10. The IPC synthase gene of *A. fumigatus* is designated herein as SEQ ID NO:13. The IPC synthase gene of *A. nidulans* is designated herein as SEQ ID NO:16. There are no intervening sequences in these genes. The IPC synthase gene of C. *neoformans* is designated herein as SEQ ID NO:19 and the cDNA thereof as SEQ ID NO:20. There is one intervening sequence in SEQ ID NO:19, from base pair 1888 through base pair 1939. Those skilled in the art will recognize that owing to the degeneracy of the genetic code (i.e. 64 codons which encode 20 amino acids), numerous "silent" substitutions of nucleotide base pairs could be introduced into these sequences without altering the identity of the encoded amino acid(s) or protein products. All such substitutions are intended to be within the scope of the invention. The genes disclosed and contemplated herein are useful for expressing the protein encoded thereby, in vitro or in a recombinant host cell.

Also contemplated is the use of said genes and fragments thereof as molecular hybridization probes for the identification and isolation of homologous genes.

Also contemplated by the present invention are nucleic acids that hybridize under high stringency conditions to the nucleic acid sequences disclosed herein.

Also contemplated are nucleic acids that are at least 70% identical in sequence to a nucleic acid sequence disclosed herein.

### Gene Isolation Procedures

Those skilled in the art will recogize that the IPC synthase genes disclosed herein may be obtained by a plurality of applicable genetic and recombinant DNA techniques including, for example, polymerase chain reaction (PCR) amplification, or de *novo* DNA synthesis. (*See e.g*., J.Sambrook et al. Molecular Cloning, 2d Ed. Chap. 14 (1989)).

Skilled artisans will recognize that the IPC synthase genes disclosed herein, or fragments thereof, could be isolated by PCR amplification of genomic DNA isolated from suitable fungal cells using oligonucleotide primers targeted to any suitable region of SEQ ID NO:1, SEQ ID NO:4, SEQ ID NO:7, SEQ ID NO:10, SEQ ID NO:13, SEQ ID NO:16, or SEQ ID NO:19. The skilled artisan understands that the choice of suitable primers may involve routine experimentation to achieve a successful outcome in a PCR amplification and that some trial and error with specific primers may be necessary. Methods for PCR amplification are widely known in the art. *See e.g.* PCR Protocols: A Guide to Method and Application, Ed. M. Innis et al., Academic Press (1990). The PCR amplification reaction comprises genomic DNA, suitable enzymes, primers, and buffers, and is conveniently carried out in a DNA Thermal Cycler (Perkin Elmer Cetus, Norwalk, CT). A positive result is determined by detecting an appropriately-sized DNA fragment following agarose gel electrophoresis.

### Protein Production Methods

One of the embodiments of the present invention relates to the purified proteins encoded by the IPC synthase genes disclosed herein, or functionally related proteins.

Skilled artisans will recognize that the proteins of the present invention can be synthesized by a variety of different methods. For example, the amino acid compounds of the invention can be made by chemical methods, well known in the art, including solid phase peptide synthesis or recombinant methods. Both methods are described in U.S. Patent 4,617,149, which hereby is incorporated by reference.

Solid phase chemical synthetic methods for polypeptides are well known in the art, and are described in general texts covering the area. *See, e.g.,* H. Dugas and C. Penney, Bioorganic Chemistry (1981) Springer-Verlag, New York, 54-92. For example, peptides may be synthesized by solid-phase methodology utilizing an Applied Biosystems 430A peptide synthesizer (Applied Biosystems, Foster City, CA) and synthesis cycles supplied by Applied Biosystems. Protected amino acids, such as t-butoxycarbonyl-protected amino acids, and other reagents are commercially available from many chemical supply houses.

The proteins of the present invention can also be produced by recombinant DNA methods using a cloned IPC synthase gene described herein. Recombinant methods are preferred if a high yield is desired. Expression of a cloned IPC synthase gene can be carried out in a variety of suitable host cells, well known to those skilled in the art. For this purpose, an IPC synthase gene is introduced into a host cell by any suitable means, well known to those skilled in the art. While chromosomal integration of the cloned IPC synthase gene is within the scope of the present invention, it is preferred that the gene be cloned into a suitable extra-chromosomally maintained expression vector so that the coding region of the IPC synthase gene is operably-linked to a constitutive or inducible promoter.

The basic steps in the recombinant production of the IPC synthase protein are:
a) constructing a natural, synthetic or semi-synthetic DNA encoding IPC synthase;
b) incorporating said DNA into an expression vector in a manner suitable for expressing an IPC synthase protein, either alone or as a fusion protein;
c) transforming an appropriate eucaryotic or prokaryotic host cell with said expression vector,
d) culturing said transformed host cell in a manner to express the IPC synthase protein; and
e) recovering membranes from said host cell and/or purifying the IPC synthase protein by any suitable means, well known to those skilled in the art.

### Expressing Recombinant IPC synthase in Procaryotic and Eucaryotic Host Cells

In general, prokaryotes are used for cloning DNA sequences and for constructing the vectors of the present invention. Prokaryotes are also employed in the production of the IPC synthase protein. For example, the *Escherichia coli* K12 strain 294 (ATCC No. 31446) is particularly useful for expressing heterologous proteins in a procaryotic host. Other strains of *E. coli,* bacilli such as *Bacillus subtilis,* enterobacteriaceae such as *Salmonella typhimurium* or *Serratia marcescans,* various Pseudomonas species and other bacteria, such as *Streptomyces,* may also be employed as host cells in cloning and expressing the recombinant proteins of this invention.

Promoters that are suitable for driving expression of genes in prokaryotes include b-lactamase [*e.g*. vector pGX2907, ATCC 39344, contains a replicon and b-lactamase gene], lactose systems [Chang et al., Nature_(London), 275:615 (1978); Goeddel et al., Nature (London), 281:544 (1979)], alkaline phosphatase, and the tryptophan (trp) promoter system [vector pATH1 (ATCC 37695) which is designed to facilitate expression of an open reading frame as a trpE fusion protein under the control of the trp promoter]. Hybrid promoters such as the tac promoter (isolatable from plasmid pDR540, ATCC-37282) are also suitable. Still other bacterial promoters, whose nucleotide sequences are generally known, enable one of skill in the art to ligate such promoter sequences to DNA encoding the proteins of the instant invention using linkers or adapters to supply any required restriction sites. Promoters for use in bacterial systems also will contain a Shine-Dalgarno sequence operably-linked to the DNA encoding the desired polypeptides. These examples are illustrative rather than limiting.

The proteins of this invention may be synthesized either by direct expression or as a fusion protein comprising the protein of interest as a translational fusion with another protein or peptide, which may be removable by enzymatic or chemical cleavage. It is often observed in the production of certain peptides in recombinant systems that expression as a fusion protein prolongs the lifespan, increases the yield of the desired peptide, or provides a convenient means of purifying the protein. A variety of peptidases (e.g. enterokinase and thrombin) which cleave a polypeptide at specific sites or digest the peptides from the amino or carboxy termini (e.g. diaminopeptidase) of the peptide chain are known. Furthermore, particular chemicals (e.g. cyanogen bromide) will cleave a polypeptide chain at specific sites. The skilled artisan will appreciate the modifications necessary to the amino acid sequence (and synthetic or semi-synthetic coding sequence if recombinant means are employed) to incorporate site-specific internal cleavage sites. *See e.g.,* P. Carter, "Site Specific Proteolysis of Fusion Proteins", Chapter 13, in Protein Purification: From Molecular Mechanisms to Large Scale Processes, American Chemical Society, Washington, D.C. (1990).

In addition to prokaryotes, mammalian host cells and eucaryotic microbes, such as yeast, may also be used to express the proteins of this invention. The simple eucaryote *Saccharomyces cerevisiae* is the most commonly used eucaryotic microorganism, although a number of other yeasts, such as *Kluyveromyces lactis,* are also suitable. For expression in *Saccharomyces,* the plasmid YRp7 (ATCC-40053), for example, may be used. *See, e.g.,* D. Stinchcomb *et al., Nature,* 282:39 (1979); J. Kingsman *et al., Gene,* 7:141 (1979); S. Tschemper *et al., Gene,* 10:157 (1980). Plasmid YRp7 contains the TRP1 gene which provides a selectable marker for use in a trpl auxotrophic mutant.

### Purification of Recombinantly-Produced IPC synthase

An expression vector carrying a cloned IPC synthase gene or cDNA from any of the fungi disclosed herein (SEQ ID NO:1, SEQ ID NO:4, SEQ ID NO:7, SEQ ID NO:10, SEQ ID NO:13, SEQ ID NO:16, SEQ ID NO:19, or SEQ ID NO:20) is transformed or transfected into a suitable host cell using standard methods. Cells which contain the vector are propagated under conditions suitable for expression of the IPC synthase protein. If an IPC synthase gene is under the control of an inducible promoter then growth conditions would incorporate the appropriate inducer. The recombinantly-produced IPC synthase protein may be purified from cellular extracts of transformed cells by any suitable means. In a preferred method for protein purification, an IPC synthase gene used in transforming a host cell is modified at the 5' end to incorporate several histidine residues at the amino terminus of the encoded IPC synthase protein. This "histidine tag" enables a single-step protein purification method referred to as "immobilized metal ion affinity chromatography" (IMAC), essentially as described in U.S. Patent 4,569,794, which hereby is incorporated by reference. The IMAC method enables rapid isolation of substantially pure protein.

IPC synthase activity can be detected in membranes from recombinant cells transformed with the genes disclosed herein, or in the membranes of non-transformed fungal cells that express said genes. Said membranes are a useful source of IPC synthase activity and can be used as a reagent in an assay for IPC synthase activity.

Other embodiments of the present invention relate to isolated nucleic acid sequences which encode SEQ ID NO:2, SEQ ID NO:5, SEQ ID NO:8, SEQ ID NO:11, SEQ ID NO:14, SEQ ID NO:17, or SEQ ID NO:21 or fragments thereof. As skilled artisans will recognize, the amino acid compounds of the invention can be encoded by a multitude of different nucleic acid sequences due to the degeneracy of the genetic code. Because these alternative nucleic acid sequences would encode the same amino acid sequences, the present invention further comprises these alternate nucleic acid sequences.

The IPC synthase genes comprising the present invention may be produced using synthetic methods well known in the art. *See, e.g.,* E.L. Brown, R. Belagaje, M.J. Ryan, and H.G. Khorana, Methods in Enzymology, 68:109-151 (1979). The DNA segments corresponding to an IPC synthase gene could be generated using a conventional DNA synthesizing apparatus, such as the Applied Biosystems Model 380A or 380B DNA synthesizers (Applied Biosystems, Inc., 850 Lincoln Center Drive, Foster City, CA 94404) which employ phosphoramidite chemistry. Alternatively, phosphotriester chemistry may be employed to synthesize the nucleic acids of this invention. *[See, e.g.,* M.J. Gait, ed., Oligonucleotide Synthesis, A Practical Approach, (1984).]

In an alternative method, IPC synthase DNA sequences comprising a portion or all of SEQ ID NO:1, SEQ ID NO:4, SEQ ID NO:7, SEQ ID NO:10, SEQ ID NO:13, SEQ ID NO:16, SEQ ID NO:19, or SEQ ID NO:20 can be generated from fungal genomic DNA using suitable oligonucleotide primers complementary to these sequences or region therein, utilizing the polymerase chain reaction as described in U.S. Patent No. 4,889,818, which is incorporated herein by reference. Protocols for performing the PCR are disclosed in, PCR Protocols: A Guide to Method and Applications, Ed. Michael A. Innis et al., Academic Press, Inc. (1990), which hereby is incorporated by reference.

The ribonucleic acids of the present invention may be prepared using polynucleotide synthetic methods discussed *supra,* or they may be prepared enzymatically using RNA polymerases to transcribe a DNA template.

The most preferred system for preparing the ribonucleic acids of the present invention employs the RNA polymerase from the bacteriophage T7 or bacteriophage SP6. These RNA polymerases are highly specific and require the insertion of bacteriophage-specific sequences at the 5' end of the template to be transcribed. *See,* J. Sambrook, et al., *supra,* at 18.82-18.84.

This invention also provides nucleic acids, RNA or DNA, which are complementary to SEQ ID NO:1, SEQ ID NO:3, SEQ ID NO:4, SEQ ID NO:6, SEQ ID NO:7, SEQ ID NO:9, SEQ ID NO:10, SEQ ID NO:12, SEQ ID NO:13, SEQ ID NO:15, SEQ ID NO:16, SEQ ID NO:18, SEQ ID NO:19, SEQ ID NO:20, or SEQ ID NO:22.

The present invention also provides probes and primers useful for molecular biology techniques. A compound that is SEQ ID NO:1, SEQ ID NO:3, SEQ ID NO:4, SEQ ID NO:6, SEQ ID NO:7, SEQ ID NO:9, SEQ ID NO:10, SEQ ID NO:12, SEQ ID NO:19, SEQ ID NO:20, or SEQ ID NO:22, or a complementary sequence thereof, or a fragment thereof, and which is at least 15 base pairs in length, and which will selectively hybridize to *C. glabrata, C. krusei, C.* *parapsilosis, C. tropicalis* or *C. neoformans* DNA or mRNA encoding IPC synthase, is provided. Preferably, the compound is DNA.

The probes and primers contemplated herein can be prepared enzymatically by well known methods *(See e.g.* Sambrook *et al. supra).*

Another aspect of the present invention relates to recombinant DNA cloning vectors and expression vectors comprising the nucleic acids described and contemplated herein. Many of the vectors encompassed within this invention are described above. The preferred nucleic acid vectors are those which comprise DNA. The most preferred recombinant DNA vectors comprise the isolated DNA sequences SEQ ID NO:1, SEQ ID NO:4, SEQ ID NO:7, SEQ ID NO:10, SEQ ID NO:13, SEQ ID NO:16, SEQ ID NO:19, or SEQ ID NO:20.

The skilled artisan understands that choosing the most appropriate cloning vector or expression vector depends upon a number of factors including the availability of appropriate restriction enzyme sites, the type of host cell into which the vector is to be transfected or transformed, the purpose of the transfection or transformation (e.g., stable transformation as an extrachromosomal element, or integration into the host chromosome), the presence or absence of readily assayable or selectable markers (e.g., antibiotic resistance markers, metabolic markers, or the like), and the number of copies of the gene to be present in the host cell.

Vectors suitable to carry the nucleic acids of the present invention include RNA viruses, DNA viruses, lytic bacteriophages, lysogenic bacteriophages, stable bacteriophages, plasmids, viroids, and the like. The most preferred vectors are plasmids.

When preparing an expression vector the skilled artisan understands that there are many variables to be considered, for example, whether to use a constitutive or inducible promoter. Inducible promoters are preferred because they may be the basis for high level and regulatable expression of an operably-linked gene. The skilled artisan will recognize a number of inducible promoters and inducers, for example, carbon source, metal ions, heat, and others known to the skilled artisan. The practitioner also understands that the amount of nucleic acid or protein to be produced dictates, in part, the selection of the expression system. The addition of certain nucleotide sequences, such as a sequence encoding a signal peptide preceding the coding sequence, is useful to direct localization of the resulting polypeptide.

Host cells harboring the nucleic acids disclosed herein are also provided by the present invention. A preferred host is *E. coli,* into which has been transfected or transformed a vector that comprises a nucleic acid of the present invention.

The present invention also provides a method for constructing a recombinant host cell capable of expressing SEQ ID NO:2, SEQ ID NO:5, SEQ ID NO:8, SEQ ID NO:11, SEQ ID NO:14, SEQ ID NO:17, or SEQ ID NO:21 said method comprising transforming a host cell with a recombinant DNA vector that comprises an isolated DNA sequence that encodes one of these sequences. Suitable host cells include any strain of *E. coli* or fungal cell that can accomodate high level expression of a gene(s) introduced by transformation or transfection. Preferred vectors for expression are those that comprise SEQ ID NO:1, SEQ ID NO:4, SEQ ID NO:7, SEQ ID NO:10, SEQ ID NO:13, SEQ ID NO:16, SEQ ID NO:19, or SEQ ID NO:20. Transformed host cells may be cultured under conditions well known to skilled artisans such that SEQ ID NO:2, SEQ ID NO:5, SEQ ID NO:8, SEQ ID NO:11, SEQ ID NO:14, SEQ ID NO:17, or SEQ ID NO:21 is expressed, thereby producing fungal IPC synthase protein in a recombinant host cell.

For the purpose of identifying or developing antifungal compounds, it would be desirable to determine those agents which inhibit IPC synthase activity. A method for determining whether a substance will inhibit the enzymatic reaction catalyzed by IPC synthase comprises contacting a source of IPC synthase activity (e.g. membrane preparation from a cell that expresses IPC synthase), or a purified IPC synthase protein, or fragment exhibiting said synthase activity, with a test substance and monitoring IPC synthase activity by any suitable means.

The instant invention provides such a screening system useful for discovering agents which inhibit an IPC synthase, said screening system comprising the steps of:
a) preparing a source of IPC synthase, or IPC synthase protein, or subunit thereof;
b) exposing said IPC synthase or source thereof to a test inhibitor;
c) introducing substrate; and
d) quantifying the loss of activity of said IPC synthase.

Utilization of the screening system described above provides a means to determine compounds that may interfere with fungal sphingolipid biosynthesis. This screening system may be adapted to automated procedures such as a PANDEX® (Baxter-Dade Diagnostics) system allowing for efficient high-volume screening of potential therapeutic agents.

In such a screening protocol IPC synthase or subunit thereof is prepared as described herein, preferably using recombinant DNA technology. Alternatively, the reaction can be carried out using membranes from cells that express ICP synthase, as a source of IPC synthase activity. Preferably, the cells are recombinant cells that incorporate a recombinantly-expressed IPC synthase into the cell membrane. Most preferably, the recombinant cells are yeast cells. A sample of a test compound is then introduced into a reaction vessel containing IPC synthase activity, followed by the addition of enzyme substrate. Alternatively, substrate may be added simultaneously with the test compound.

The following examples more fully describe the present invention. Those skilled in the art will recognize that the particular reagents, equipment, and procedures described are merely illustrative and are not intended to limit the present invention in any manner.

### EXAMPLE 1

### Construction of a DNA Vector for Expressing the C. glabrata IPC synthase Gene in Homologous or Heterologous Host

An expression vector suitable for expressing the IPC synthase gene of *C. glabrata* (SEQ ID NO:1) in *E. coli* contains an origin of replication (Ori), an ampicillin resistance gene (Amp) useful for selecting cells that have incorporated the vector following tranformation. The vector also includes the T7 promoter and T7 terminator sequences in operable linkage to the coding region of the IPC synthase gene. Parent plasmid pET11A (obtained from Novogen, Madison, WI) is linearized by digestion with appropriate endonucleases and ligated to a DNA fragment comprising the coding region of the *C. glabrata* IPC synthase gene.

The IPC synthase gene ligated into the expression vector is modified at the 5' end (amino terminus of encoded protein) in order to simplify purification of the encoded IPC synthase protein product. For this purpose, an oligonucleotide encoding 8 histidine residues and a factor Xa cleavage site is inserted after the ATG start codon at nucleotide positions 1 to 3 of SEQ ID NO:1. Placement of the histidine residues at the amino terminus of the encoded protein enables the IMAC one-step protein purification procedure *(See* below).

### EXAMPLE 2

### Expression of C.glabrata IPC synthase Gene in Echerichia coli and Purification of IPC synthase Enzyme

A plasmid from Example 1 is transformed into *E. coli* BL21 *(DE3) (hsdS gal* lcIts857 *ind*1*Sam*7*nin*5*lac*UV5-T7gene 1) using standard methods *(See e.g.* Sambrook *et al. Supra).* Transformants, selected for resistance to ampicillin, are chosen at random and tested for the presence of the vector by agarose gel electrophoresis using quick plasmid preparations. Colonies that contain the vector are grown, processed, and the protein encoded by the IPC synthase gene is purified by immobilized metal ion affinity chromatography (IMAC), essentially as described in US Patent 4,569,794, the entire contents of which is hereby incorporated by reference.

Briefly, the IMAC column is prepared as follows. A metal-free chelating resin (e.g. SEPHAROSE 6B IDA, Pharmacia) is washed in distilled water to remove preservative substances and infused with a suitable metal ion [e.g. Ni(II), Co(II), or Cu(II)] by adding a 50 mM metal chloride or metal sulfate aqueous solution until about 75% of the interstitial spaces of the resin is saturated with colored metal ion. The column is then ready to receive a crude cellular extract containing the IPC synthase protein product encoded by the vector.

After removing unbound proteins and other materials by washing the column with suitable buffer, pH 7.5, the bound protein is eluted in buffer at pH 4.3 essentially as described in U.S. Patent 4,569,794.

### EXAMPLE 3

### Biochemical Assay for Inhibitors of IPC synthase Using Fungal Membrane Preparations

The activity of the IPC synthase enzyme is assayed by preparing membranes from *C. glabrata,* for example, and using said membranes as a source of IPC synthase activity.

A suitable, rich medium, for example YEPD, is innoculated with a culture and allowed to grow overnight at room temperature with vigorous shaking. About 250 ml of fresh medium containing 20 ug/ml myo-inositol is innoculated with the overnight culture, and grown overnight at 30° C. Cells are harvested by centrifugation and resuspended in ice cold 50 mM potassium phosphate buffer, pH 7. Cells are washed twice in the same buffer and then resuspended in the same buffer containing 5 mM dithiothreitol (DTT), 1 ug/ml aprotinin, 0.6 uM leupeptin, 1 mM PMSF, and 1 ug/ml pepstatin A. Cells are ruptured using glass beads in a procedure that involves 5 successive vortexings each for 30 seconds followed by 2 to 5 minute intervals of rest on ice. Membranes are pelleted by centrifugation at 100,000 x g for 1 hour at 4° C. The pellet is resuspended in cold buffer containing DTT and protease inhibitors and disrupted further by Dounce homogenization with 5 to 6 strokes on ice. Membranes are mixed with glycerol to a final concentration of 33% and stored frozen at - 80° C. For use as "protein stock," thaw and dilute to 4.8 mg/ml in 0.05 M potassium phosphate(Kpi) buffer, pH 7.0 on ice.

IPC synthase is assayed by any suitable method. For example:

| Final Assay Conditions: |
|---|
| 5-50 uM NBD-C6-ceramide (from Molecular Probes) |
| 1 mM phosphatidylinositol (Sigma-soybean) |
| 2 mM CHAPS |
| 50 mM potassium phosphate buffer, pH 7.0 |
| Up to 2.5% organic co-solvent |
| Protein to 1.2 mg/ml |
| Final Vol. 100 ul |

| 2X Master Mix (for 15 samples) |
|---|
| 46 ul of 1 mg/ml NBD-C6-ceramide in MeOH |
| 142 ul of 10 mg/ml phosphatidylinositol in |
| CHCl₃ |
| Dry under vacuum |
| Redissolve by adding in order |
| 160 ul 20 mM CHAPS |
| 560 ul deionized water |
| 80 ul 0.5 M Kpi buffer, pH 7.0 |
| Sonicate 5 minutes. |

Aliquot 50 ul of 2X master mix to screw cap microfuge at room temperature. Add 20 ul of 0.05 M Kpi buffer pH 7.0 to each sample followed by 5 ul of test compound or extract. The reaction is initiated by adding 25 ul of protein stock and incubating for 1 hour in a 30° C water bath. Reactions are terminated by adding 900 ul of cold absolute methanol. Samples are stored at -20° C for 1 hour and centrifuged at 14000 x g at 4° C for 30 minutes.

The reaction is monitored by HPLC on a Beckman Ultrasphere-ODS 5 um, 150 x 4 mm column. The chromatogram is developed in 87% MeOH/13% 50 mM TEAP, pH 5.8, at a flow rate of 1 ml/min at room temperature through a Beckman Model 157 Fluorescence detector, using NBD-C₆-phosphatidylcholine as an internal standard.

Inhibition studies are carried out using the same reaction conditions described in the preceding paragraph. Compounds to be studied for inhibitory activity are added to final concentrations of between 1 pM and 10 mM.

### EXAMPLE 4

### Expression of C.parapsilosis (SEQ ID NO:7) IPC synthase Gene in S. cerevisiae

A yeast/E.coli shuttle vector suitable for expressing the *C. parapsilosis* IPC synthase gene (SEQ ID NO:7) in *S. cerevisiae is* constructed in parent plasmid YEp351 (See J.Hill *et.al., Yeast,* 2, 163-167, 1986), which contains the multiple cloning region of pUC18, the Amp^{R} gene for selection in E.coli, 2µ replicon, and the LEU2 gene for selection in yeast. A fragment containing the *C.parapsilosis* IPC synthase gene is prepared by PCR amplification. Suitable primers to the 5' and 3' ends of the coding region disclosed in SEQ ID NO:7 are constructed to contain BamH1 cloning sites in addition to IPC synthase coding information. After PCR amplification of *C.parapsilosis* genomic DNA, the amplified fragment is purified by any sutiable method, for example, gel purification of an appropriately sized fragment, followed by treatment with restriction enzyme BamH1, and ligation to BamH1 digested YEp351. The recombinant plasmid carrying the IPC synthase gene is transformed into any suitable leu⁻ strain of *S. cerevisiae* and transformants selected for growth in a medium that lacks added leucine. Membranes from tranformants are prepared, and IPC synthase activity assayed as in Example 3.

## Claims

1. A substantially pure IPC synthase protein from a fungal cell other than *Saccharomyces cerevisiae.*

2. A substantially pure IPC synthase protein from a fungal cell comprising an amino acid sequence selected from the group consisting of:
a) SEQ ID NO:2;
b) SEQ ID NO:5;
c) SEQ ID NO:8;
d) SEQ ID NO:11; or
e) SEQ ID NO:21.

3. An isolated nucleic acid encoding a protein of Claim 1.

4. An isolated nucleic acid encoding a protein of Claim 2.

5. An isolated nucleic acid compound encoding a fungal IPC synthase protein of Claim 2, or fragment thereof, wherein said compound has a sequence selected from the group consisting of:
(a) SEQ ID NO:1;
(b) SEQ ID NO:3;
(c) SEQ ID NO:4;
(d) SEQ ID NO:6;
(e) SEQ ID NO:7;
(f) SEQ ID NO:9;
(g) SEQ ID NO:10;
(h) SEQ ID NO:12;
(i) SEQ ID NO:19;
(j) SEQ ID NO:20;
(k) SEQ ID NO:22;
(l) a nucleic acid compound complementary to (a), (b), (c), (d), (e), (f), (g), (h), (i), (j), or (k); and
(m) a fragment of (a), (b), (c), (d), (e), (f), (g), (h), (i), (j), (k), or (l) that is at least 18 base pairs in length and which will selectively hybridize to IPC synthase genomic DNA
(n) a nucleic acid that is at least 70% homologous to a nucleic acid disclosed in (a) - (k) .

6. A vector comprising an isolated nucleic acid compound of Claim 5.

7. A vector, as in Claim 6, wherein said isolated nucleic acid compound is SEQ ID NO:1, SEQ ID NO:4, SEQ ID NO:7, SEQ ID NO:10, SEQ ID NO:19, or SEQ ID NO:20, operably-linked to a promoter sequence.

8. A host cell containing a vector of Claim 6 or Claim 7.

9. A method for identifying inhibitory compounds of fungal IPC synthase protein activity, comprising the steps of:
a) admixing in a suitable reaction buffer
i) a source of IPC synthase protein;
ii) a suitable substrate;
iii) a test inhibitory compound;
b) measuring by any suitable means an amount of product formed; and
c) comparing the amount of product formed at step (b) with a control reaction, said control reaction comprising steps (a) (i), (a) (ii), and (b) and wherein said control reaction lacks said test inhibitory compound.

10. A method, as in Claim 9, wherein at step (a) (i) said source comprises membranes from a cell that expresses IPC synthase, and wherein said cell is a recombinant host cell that expresses a vector-borne IPC synthase gene selected from the group consisting of SEQ ID NO:1, SEQ ID NO:4, SEQ ID NO:7, SEQ ID NO:10, SEQ ID NO:13, SEQ ID NO:16, SEQ ID NO:19, and SEQ ID NO:20.


### Annex to the description

SEQUENCE LISTING

(1) GENERAL INFORMATION:

(i) APPLICANT: ELI LILLY AND COMPANY
(B) STREET: Lilly Corporate Center
(C) CITY: Indianapolis
(D) STATE: Indiana
(E) COUNTRY: United States of America
(F) ZIP: 46285

(ii) TITLE OF INVENTION: IPC Synthase Genes From Fungi

(iii) NUMBER OF SEQUENCES: 22

(iv) CORRESPONDENCE ADDRESS:
(A) ADDRESSEE: A. M. Denholm
(B) STREET: Erl Wood Manor
(C) CITY: Windlesham
(D) STATE: Surrey
(E) COUNTRY: United Kingdom
(F) ZIP: GU20 6PH

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30

(2) INFORMATION FOR SEQ ID NO:1:

(i) SEQUENCE CHARACTERISTICS:
(A) LENGTH: 2000 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(ix) FEATURE:
(A) NAME/KEY: CDS
(B) LOCATION: 501..1748

(xi) SEQUENCE DESCRIPTION: SEQ ID NO:1:

AAAACGTCAT TTTAAAATTA GGCTTTAGAT TGTAACTCAG ATGATTTTCG TCAATCCAGT 60

GTACGAAACT TGGCAAATGC TAAAACTTCA ACAAATAAAT CATCATCCCT AATACTAGTG 120

TAAATAATTT AATGTACATG GTGTTCTATT TCTAATGTAG AAATATGTAT ATGACATTAG 180

ATCAACTCAT ATTGTTCAAA ACTCTGTCAA AATAATTTAG TGAGGCAAAG GTGTCGTCCC 240

CCGAATGAAC TTGTAACTCA CCTTGAAGGA GATATTTGCA TTTACTTGAT AATCCGAAAC 300

TTTTGCAACT TAACATTTTA AAACTAGAAG TTATTTGGTA TCTGGTATTA ATTTTTATTA 360

GCTTTGCTGA TTACGACACG TTACGAAGGA AGATATTGAC GCTCAGAATT TTTATTTAGG 420

AGAAATTTAT CATAATAGAC AATATTTGCC ATTACCGATT TCTAACTAAA TATCATAATC 480

ATAGTTATTA GACAAATTCA ATG AGA CTT GGA AGA TGG TTT GCT ATT TTG 530
Met Arg Leu Gly Arg Trp Phe Ala Ile Leu
1               5                   10

AGC TCA AAA TTA TGC TCA GAT ACA GAT GTA ACT AAG TTG GAG AGA AGT 578
Ser Ser Lys Leu Cys Ser Asp Thr Asp Val Thr Lys Leu Glu Arg Ser
                15                  20                  25

CTT GAT GTA AGG GTC ACA TTA AGA CGA CTC AGG AGT TAC AGA CCC ACA 626
Leu Asp Val Arg Val Thr Leu Arg Arg Leu Arg Ser Tyr Arg Pro Thr
            30                  35                  40

ATA GGT GAC ACA GTT CAT TAT TCA TTT TTG TCA ATG ATT CTA TTG TAT 674
Ile Gly Asp Thr Val His Tyr Ser Phe Leu Ser Met Ile Leu Leu Tyr
        45                  50                  55

TCA TAT TTT GCT AAT CCA CTA CCA TTC ATT GTG AAA ACA TTG ATA GCG 722
Ser Tyr Phe Ala Asn Pro Leu Pro Phe Ile Val Lys Thr Leu Ile Ala
    60                  65                  70

ATG ATT TTA ATG ACT CTG TTT GTC GTC CCG ATG ACA TCT CAA TTT TTC 770
Met Ile Leu Met Thr Leu Phe Val Val Pro Met Thr Ser Gln Phe Phe
75                  80                  85                  90

TTT AAT GCA CTT CCA ATA TTG ACA TGG CTA ATC TTG TAT TTT ACA TCA 818
Phe Asn Ala Leu Pro Ile Leu Thr Trp Leu Ile Leu Tyr Phe Thr Ser
                95                  100                 105

TCA CAT ATT CCT AAT AGT CAT AGA CCT TCA ATT TCT GTT GAG GTG CTT 866
Ser His Ile Pro Asn Ser His Arg Pro Ser Ile Ser Val Glu Val Leu
            110                 115                 120

CCT GCC ATT GAA ACT ATC CTG TAT GGC GAT AAC TTA AGT GAG ATT TTG 914
Pro Ala Ile Glu Thr Ile Leu Tyr Gly Asp Asn Leu Ser Glu Ile Leu
        125                 130                 135

GCT GCA TGG CAA AAT ACA ATT CTA GAT ATC CTA GCT TGG ATT CCA TAT 962
Ala Ala Trp Gln Asn Thr Ile Leu Asp Ile Leu Ala Trp Ile Pro Tyr
    140                 145                 150

GGT CTG TTT CAC TTT GGC GCA CCA TTT GTG GTT GCA ATT GTT TTA TTT 1010
Gly Leu Phe His Phe Gly Ala Pro Phe Val Val Ala Ile Val Leu Phe
155                 160                 165                 170

ATA TTT GGA CCA CCG ACA GTT CTT CAG GGT TAT GCG TTT GCG TTT GGT 1058
Ile Phe Gly Pro Pro Thr Val Leu Gln Gly Tyr Ala Phe Ala Phe Gly
                175                 180                 185

TAT GTG AAT TTA TTT GGG GTG TTA TTC CAA AAC ATA TTT CCC GCC GCG 1106
Tyr Val Asn Leu Phe Gly Val Leu Phe Gln Asn Ile Phe Pro Ala Ala
            190                 195                 200

GCT CCT TGG TAT AAA ATA CAT TAT GGA CTC CAA GTT GCA AAT TAC AGT 1154
Ala Pro Trp Tyr Lys Ile His Tyr Gly Leu Gln Val Ala Asn Tyr Ser
        205                 210                 215

ATC AAA GGT ATG CCT GGT GGA CTT TCT AGA ATT GAT GAT CTT TTG GGT 1202

Ile Lys Gly Met Pro Gly Gly Leu Ser Arg Ile Asp Asp Leu Leu Gly
    220                 225                 230

GTG AAT TTA TAT TCA AGT GGT TTT AAG AAC TCT GCT GTC GTT TTT GGA      1250
Val Asn Leu Tyr Ser Ser Gly Phe Lys Asn Ser Ala Val Val Phe Gly
235                 240                 245                 250

GCT TTT CCT TCT CTT CAT TCT GCA TGT GCA ACT ATG GAA GCA CTC TTC      1298
Ala Phe Pro Ser Leu His Ser Ala Cys Ala Thr Met Glu Ala Leu Phe
                255                 260                 265

TTC TCA TAT TGT TTT CCA AGC TTG ACA CCG CTC TTT ATT TTC TAC GTT      1346
Phe Ser Tyr Cys Phe Pro Ser Leu Thr Pro Leu Phe Ile Phe Tyr Val
            270                 275                 280

TGG TGG TTA TGG TGG TCT ACA ATG TAT CTT ACT CAT CAT TAT TTT GTT      1394
Trp Trp Leu Trp Trp Ser Thr Met Tyr Leu Thr His His Tyr Phe Val
        285                 290                 295

GAT CTA ACA GCA GGT TCT GTT TTG GCT TAT TTC ATA TTT CAA TAT ACC      1442
Asp Leu Thr Ala Gly Ser Val Leu Ala Tyr Phe Ile Phe Gln Tyr Thr
    300                 305                 310

AAA TAC ACA CAT CTA CCT ATT GTT GAT CCA AAT CTG CTC TTC CGT TGG      1490
Lys Tyr Thr His Leu Pro Ile Val Asp Pro Asn Leu Leu Phe Arg Trp
315                 320                 325                 330

TCC TAC GAC AGT ATT GAA TTT TAC GAT GTT AAG AAC AAT GAC CCT TTA      1538
Ser Tyr Asp Ser Ile Glu Phe Tyr Asp Val Lys Asn Asn Asp Pro Leu
                335                 340                 345

TCC ATT ACA AAT AAC GAT ATA GAG AGT ATA CCT CTA AAC ACC ATT GAA      1586
Ser Ile Thr Asn Asn Asp Ile Glu Ser Ile Pro Leu Asn Thr Ile Glu
            350                 355                 360

CTA GAT ACA GAA GAA GTG TTC AAT ATT GAG CGG GAA AAA TCT AGG ACA      1634
Leu Asp Thr Glu Glu Val Phe Asn Ile Glu Arg Glu Lys Ser Arg Thr
        365                 370                 375

CCA GAA TTG TCA CAT GCA GTG TCT TCA TCT TCT CTA GCT AAC GGT CGC      1682
Pro Glu Leu Ser His Ala Val Ser Ser Ser Ser Leu Ala Asn Gly Arg
    380                 385                 390

TCT CAA ACA TAT CCA AAT ACT GGA AAT ACA TTC AAA TCC AAG ACA AGA      1730
Ser Gln Thr Tyr Pro Asn Thr Gly Asn Thr Phe Lys Ser Lys Thr Arg
395                 400                 405                 410

CTG AGT AAA CTG GTC ATT TAAAACCAGT ATTTATAAAT AGAGACTATC              1778
Leu Ser Lys Leu Val Ile
                415

TTCTTCTACT GTACATACAA TGATCTTTCT ATATTCTAAC GAAATCAATA AGTGCATACT    1838

ATATTCATTT AACATGATCA ATATTATTTA ACAGATCCAT TAGGTGCTGG CTTCCCAAGA    1898

TAGTTACCAA AACGATAATT TTTTCTCAAA ATTTGCCCTT TGAGAAAAAC CTCCGTCTAG    1958

TCACCCCTTA CCACACAGCA CAAACTGAAA ATCGATGGGA TA                        2000

(2) INFORMATION FOR SEQ ID NO:2:

        (i) SEQUENCE CHARACTERISTICS:
            (A) LENGTH: 416 amino acids
            (B) TYPE: amino acid

(D) TOPOLOGY: linear

(ii) MOLECULE TYPE: protein

(xi) SEQUENCE DESCRIPTION: SEQ ID NO:2:

Met Arg Leu Gly Arg Trp Phe Ala Ile Leu Ser Ser Lys Leu Cys Ser
1               5                   10                  15

Asp Thr Asp Val Thr Lys Leu Glu Arg Ser Leu Asp Val Arg Val Thr
            20                  25                  30

Leu Arg Arg Leu Arg Ser Tyr Arg Pro Thr Ile Gly Asp Thr Val His
        35                  40                  45

Tyr Ser Phe Leu Ser Met Ile Leu Leu Tyr Ser Tyr Phe Ala Asn Pro
    50                  55                  60

Leu Pro Phe Ile Val Lys Thr Leu Ile Ala Met Ile Leu Met Thr Leu
65                  70                  75                  80

Phe Val Val Pro Met Thr Ser Gln Phe Phe Phe Asn Ala Leu Pro Ile
                85                  90                  95

Leu Thr Trp Leu Ile Leu Tyr Phe Thr Ser Ser His Ile Pro Asn Ser
            100                 105                 110

His Arg Pro Ser Ile Ser Val Glu Val Leu Pro Ala Ile Glu Thr Ile
        115                 120                 125

Leu Tyr Gly Asp Asn Leu Ser Glu Ile Leu Ala Ala Trp Gln Asn Thr
    130                 135                 140

Ile Leu Asp Ile Leu Ala Trp Ile Pro Tyr Gly Leu Phe His Phe Gly
145                 150                 155                 160

Ala Pro Phe Val Val Ala Ile Val Leu Phe Ile Phe Gly Pro Pro Thr
                165                 170                 175

Val Leu Gln Gly Tyr Ala Phe Ala Phe Gly Tyr Val Asn Leu Phe Gly
            180                 185                 190

Val Leu Phe Gln Asn Ile Phe Pro Ala Ala Ala Pro Trp Tyr Lys Ile
        195                 200                 205

His Tyr Gly Leu Gln Val Ala Asn Tyr Ser Ile Lys Gly Met Pro Gly
    210                 215                 220

Gly Leu Ser Arg Ile Asp Asp Leu Leu Gly Val Asn Leu Tyr Ser Ser
225                 230                 235                 240

Gly Phe Lys Asn Ser Ala Val Val Phe Gly Ala Phe Pro Ser Leu His
                245                 250                 255

Ser Ala Cys Ala Thr Met Glu Ala Leu Phe Phe Ser Tyr Cys Phe Pro
            260                 265                 270

Ser Leu Thr Pro Leu Phe Ile Phe Tyr Val Trp Trp Leu Trp Trp Ser
        275                 280                 285

Thr Met Tyr Leu Thr His His Tyr Phe Val Asp Leu Thr Ala Gly Ser
    290                 295                 300

Val Leu Ala Tyr Phe Ile Phe Gln Tyr Thr Lys Tyr Thr His Leu Pro
305                 310                 315                 320

Ile Val Asp Pro Asn Leu Leu Phe Arg Trp Ser Tyr Asp Ser Ile Glu
                325                 330                 335

Phe Tyr Asp Val Lys Asn Asn Asp Pro Leu Ser Ile Thr Asn Asn Asp
            340                 345                 350

Ile Glu Ser Ile Pro Leu Asn Thr Ile Glu Leu Asp Thr Glu Glu Val
        355                 360                 365

Phe Asn Ile Glu Arg Glu Lys Ser Arg Thr Pro Glu Leu Ser His Ala
    370                 375                 380

Val Ser Ser Ser Ser Leu Ala Asn Gly Arg Ser Gln Thr Tyr Pro Asn
385                 390                 395                 400

Thr Gly Asn Thr Phe Lys Ser Lys Thr Arg Leu Ser Lys Leu Val Ile
                405                 410                 415

(2) INFORMATION FOR SEQ ID NO:3:

     (i) SEQUENCE CHARACTERISTICS:
          (A) LENGTH: 1248 base pairs
          (B) TYPE: nucleic acid
          (C) STRANDEDNESS: single
          (D) TOPOLOGY: linear

    (ii) MOLECULE TYPE: mRNA

   (iii) HYPOTHETICAL: NO

    (iv) ANTI-SENSE: NO

    (xi) SEQUENCE DESCRIPTION: SEQ ID NO:3:

AUGAGACUUG GAAGAUGGUU UGCUAUUUUG AGCUCAAAAU UAUGCUCAGA UACAGAUGUA      60

ACUAAGUUGG AGAGAAGUCU UGAUGUAAGG GUCACAUUAA GACGACUCAG GAGUUACAGA     120

CCCACAAUAG GUGACACAGU UCAUUAUUCA UUUUUGUCAA UGAUUCUAUU GUAUUCAUAU     180

UUUGCUAAUC CACUACCAUU CAUUGUGAAA ACAUUGAUAG CGAUGAUUUU AAUGACUCUG     240

UUUGUCGUCC CGAUGACAUC UCAAUUUUUC UUUAAUGCAC UUCCAAUAUU GACAUGGCUA     300

AUCUUGUAUU UUACAUCAUC ACAUAUUCCU AAUAGUCAUA GACCUUCAAU UUCUGUUGAG     360

GUGCUUCCUG CCAUUGAAAC UAUCCUGUAU GGCGAUAACU UAAGUGAGAU UUUGGCUGCA     420

UGGCAAAAUA CAAUUCUAGA UAUCCUAGCU UGGAUUCCAU AUGGUCUGUU UCACUUUGGC     480

GCACCAUUUG UGGUUGCAAU UGUUUUAUUU AUAUUUGGAC CACCGACAGU UCUUCAGGGU     540

UAUGCGUUUG CGUUUGGUUA UGUGAAUUUA UUUGGGGUGU UAUUCCAAAA CAUAUUUCCC     600

GCCGCGGCUC CUUGGUAUAA AAUACAUUAU GGACUCCAAG UUGCAAAUUA CAGUAUCAAA     660

GGUAUGCCUG GUGGACUUUC UAGAAUUGAU GAUCUUUUGG GUGUGAAUUU AUAUUCAAGU     720

GGUUUUAAGA ACUCUGCUGU CGUUUUUGGA GCUUUUCCUU CUCUUCAUUC UGCAUGUGCA     780

ACUAUGGAAG CACUCUUCUU CUCAUAUUGU UUUCCAAGCU UGACACCGCU CUUUAUUUUC 840

UACGUUUGGU GGUUAUGGUG GUCUACAAUG UAUCUUACUC AUCAUUAUUU UGUUGAUCUA 900

ACAGCAGGUU CUGUUUUGGC UUAUUUCAUA UUUCAAUAUA CCAAAUACAC ACAUCUACCU 960

AUUGUUGAUC CAAAUCUGCU CUUCCGUUGG UCCUACGACA GUAUUGAAUU UUACGAUGUU 1020

AAGAACAAUG ACCCUUUAUC CAUUACAAAU AACGAUAUAG AGAGUAUACC UCUAAACACC 1080

AUUGAACUAG AUACAGAAGA AGUGUUCAAU AUUGAGCGGG AAAAAUCUAG GACACCAGAA 1140

UUGUCACAUG CAGUGUCUUC AUCUUCUCUA GCUAACGGUC GCUCUCAAAC AUAUCCAAAU 1200

ACUGGAAAUA CAUUCAAAUC CAAGACAAGA CUGAGUAAAC UGGUCAUU 1248

(2) INFORMATION FOR SEQ ID NO:4:

(i) SEQUENCE CHARACTERISTICS:
(A) LENGTH: 2000 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(ix) FEATURE:
(A) NAME/KEY: CDS
(B) LOCATION: 300..1739

(xi) SEQUENCE DESCRIPTION: SEQ ID NO:4:

ACAGTAAAAA GAAATTGCGC TGCATGAAAC TTTTTTTTTT TTTTTTTTCG GTGTTTCTTC 60

TCGTTTTTCT CGTTTTTCTC GTTTTTCTCG GTTTCTTTTG AGAATGTAAT GAAATTTGGA 120

AACTGTACAA ACATTCTCAG TGTTGATATT AACAATAATA ACCACATTGA CATTGACAAC 180

ATCATTATTA GGTTCTTTCC ATTTGTATTA TTTCCATCTC GGACACGTCA GCTATAGACC 240

GTCGACACTC CCTATTCCCT GCACCAACTA GGAAGAACAG GAAGAACAAG AAGAACAGA 299

ATG CTC TCG CTC CCG GCC CTT ATT AGG CGC CTG TAT TCC TCC TTT ATT 347
Met Leu Ser Leu Pro Ala Leu Ile Arg Arg Leu Tyr Ser Ser Phe Ile
1               5                   10                  15

TCG GCA AGA GAA AAG GGT AAT TCA TTG CCT GCA TTA AAG ACA TCC TTT 395
Ser Ala Arg Glu Lys Gly Asn Ser Leu Pro Ala Leu Lys Thr Ser Phe
            20                  25                  30

AAC CCC CTC CCT AAC TTC AAG ACC TTC CCG GAG AGA CTG GTC TCG GCA 443
Asn Pro Leu Pro Asn Phe Lys Thr Phe Pro Glu Arg Leu Val Ser Ala
        35                  40                  45

TCC ATA TCC AAT GTG GTC CAT TTC ACG ATT ATC TTT CTT GTC TTT GTC 491
Ser Ile Ser Asn Val Val His Phe Thr Ile Ile Phe Leu Val Phe Val
    50                  55                  60

TTC TCC ATC TCA ATA ATG CCT GTA CCC CTA CTT GTG AAA GCC ATT CTT 539

Phe Ser Ile Ser Ile Met Pro Val Pro Leu Leu Val Lys Ala Ile Leu
65                  70                  75                  80

ATC ATT GTC TTG ATT TAC ATC AAC TTT GCT CCA ATT TTC GCC CAG TTC     587
Ile Ile Val Leu Ile Tyr Ile Asn Phe Ala Pro Ile Phe Ala Gln Phe
                85                  90                  95

TTT AAC GGC GGC TTG CCG ATC CTC TCA TGG GTC ATT TTG TTC TTT AAC     635
Phe Asn Gly Gly Leu Pro Ile Leu Ser Trp Val Ile Leu Phe Phe Asn
            100                 105                 110

TCA AAG AAC ATA CCT CTG TCA ATG AAG AGA CCC ATC TCC GTC AAG GTC     683
Ser Lys Asn Ile Pro Leu Ser Met Lys Arg Pro Ile Ser Val Lys Val
        115                 120                 125

TTG CCC GCA TTG GAG ACA ATC TTC TAT GGC GAT AAT TTA TCT GAA ATC     731
Leu Pro Ala Leu Glu Thr Ile Phe Tyr Gly Asp Asn Leu Ser Glu Ile
    130                 135                 140

TTG GCT TCT CAT ACA AAC ACT TTC TTG GAC TTG TTT GCT TGG TTC CCA     779
Leu Ala Ser His Thr Asn Thr Phe Leu Asp Leu Phe Ala Trp Phe Pro
145                 150                 155                 160

TAT GGT ATA GTC CAT TTC TCC CTG CCC TTT GTT GTG GCA GCC CTT ATT     827
Tyr Gly Ile Val His Phe Ser Leu Pro Phe Val Val Ala Ala Leu Ile
                165                 170                 175

TTT TTA TTT GCC CCC CCT AAA ACC TTA CCC CAA TTT TGT TGG TCC TTT     875
Phe Leu Phe Ala Pro Pro Lys Thr Leu Pro Gln Phe Cys Trp Ser Phe
            180                 185                 190

GGT TAT ATG AAT TTG GTT GGC GTC ATT ATC CAA AAC TTG GTT TTC TCA     923
Gly Tyr Met Asn Leu Val Gly Val Ile Ile Gln Asn Leu Val Phe Ser
        195                 200                 205

TGT GCT CCA CCA TGG TAT AAA GTG CTG CAT GGG TTG GAC AAG GCA AAC     971
Cys Ala Pro Pro Trp Tyr Lys Val Leu His Gly Leu Asp Lys Ala Asn
    210                 215                 220

TAT TCA ATG AAG GGA TCG CCA GGA GGA TTG GGT AGA ATC GAT GGA CTG     1019
Tyr Ser Met Lys Gly Ser Pro Gly Gly Leu Gly Arg Ile Asp Gly Leu
225                 230                 235                 240

TTT GGT TTC GAT ATG TAC ACC TCG GGT TTC ACC AAT TCT CCC TTG ATT     1067
Phe Gly Phe Asp Met Tyr Thr Ser Gly Phe Thr Asn Ser Pro Leu Ile
                245                 250                 255

TTC GGT GCA ATG CCC TCC TTG CAT TCG GGA TGT TCA ACT ATG GAC GCA     1115
Phe Gly Ala Met Pro Ser Leu His Ser Gly Cys Ser Thr Met Asp Ala
            260                 265                 270

TTA TGG TTG TCT TAT CTA TTC CCT AAA TTC ACC CCT CTA TTT GTT TTC     1163
Leu Trp Leu Ser Tyr Leu Phe Pro Lys Phe Thr Pro Leu Phe Val Phe
        275                 280                 285

TAC GTT TGC TGG TTA TGG TTC TCT ACA ATG TAT TTG ACT CAT CAT TAC     1211
Tyr Val Cys Trp Leu Trp Phe Ser Thr Met Tyr Leu Thr His His Tyr
    290                 295                 300

TTC ATC GAT GTT GTT GTT GGT TCT TGT TTG GCA ATT GGA TTC TTC ACT     1259
Phe Ile Asp Val Val Val Gly Ser Cys Leu Ala Ile Gly Phe Phe Thr
305                 310                 315                 320

GCA GTC AAA TGG AGA GGC AGA TTA CCC GTA AAT GAT CTA TTT TGT AGG     1307
Ala Val Lys Trp Arg Gly Arg Leu Pro Val Asn Asp Leu Phe Cys Arg

                325                 330                 335

TGG GAC CAT GAT ACC CTG AAA TTC CAT GAC ATT TGG AAC GAG GAC CCA 1355
Trp Asp His Asp Thr Leu Lys Phe His Asp Ile Trp Asn Glu Asp Pro
            340                 345                 350

CTG AGG GAG AAC TAT CCC TTG GAC TTG GAG TCT TCA AAA GAC TTG GAA 1403
Leu Arg Glu Asn Tyr Pro Leu Asp Leu Glu Ser Ser Lys Asp Leu Glu
        355                 360                 365

AAT CAG ATA CCT TTG AGT GAT TTG AAT TCA TCA TGT GAT GAA CAA CCA 1451
Asn Gln Ile Pro Leu Ser Asp Leu Asn Ser Ser Cys Asp Glu Gln Pro
    370                 375                 380

AGT TCA CCA AAG GGA TTT AAT AGG AAC CAG AAT AAT AAC TTG GGT GAT 1499
Ser Ser Pro Lys Gly Phe Asn Arg Asn Gln Asn Asn Asn Leu Gly Asp
385                 390                 395                 400

AAG GGA CAG GGA TTA GAA CAA GTA GAC GAT CAA GGA GAA GAA TAT GAC 1547
Lys Gly Gln Gly Leu Glu Gln Val Asp Asp Gln Gly Glu Glu Tyr Asp
                405                 410                 415

AAT TAC GAT TTT GAT GAT TCT GAT GAC GAC CAA ATT GCT GAA GCT AAT 1595
Asn Tyr Asp Phe Asp Asp Ser Asp Asp Asp Gln Ile Ala Glu Ala Asn
            420                 425                 430

GAT GTG GAG AAA TCG GCT AAC AAA ACA ATA AAG AAG ACT CCA AAG TCA 1643
Asp Val Glu Lys Ser Ala Asn Lys Thr Ile Lys Lys Thr Pro Lys Ser
        435                 440                 445

TAC AAG GAA TCA TTC GAA GCA ACA TTA TCT ACA AAC TTG AAC TCA CAA 1691
Tyr Lys Glu Ser Phe Glu Ala Thr Leu Ser Thr Asn Leu Asn Ser Gln
    450                 455                 460

ATT ACA AAT TTC CCA TCT TCC ACT CAT ATA CCT CTT AAT TCC AGT CGT 1739
Ile Thr Asn Phe Pro Ser Ser Thr His Ile Pro Leu Asn Ser Ser Arg
465                 470                 475                 480

TGACACAAAC TCGGTTCTTT AATAACTACC ACTTCGCCCT TTACTTAGCG TCTTTGAAAT 1799

TATAGATCTG GAATTAGAAA TAAACTACCT TTTTTTTTGG CCAATTGGAA AAATTTAATA 1859

CAACTCTTAT CAAGTCACTC CTTTATTTTC AATCTTTTTC TTTATGTACA TGTAATTTTA 1919

CAGAGTTCAA TAGTTAATTA TTTAATTACT TATTTTCACT TTATAGTTAC ACTCTTCTCC 1979

CCACGTCCTT TTTTCTTATC A 2000

(2) INFORMATION FOR SEQ ID NO:5:

(i) SEQUENCE CHARACTERISTICS:
(A) LENGTH: 480 amino acids
(B) TYPE: amino acid
(D) TOPOLOGY: linear

(ii) MOLECULE TYPE: protein

(xi) SEQUENCE DESCRIPTION: SEQ ID NO:5:

Met Leu Ser Leu Pro Ala Leu Ile Arg Arg Leu Tyr Ser Ser Phe Ile
1               5                   10                  15

Ser Ala Arg Glu Lys Gly Asn Ser Leu Pro Ala Leu Lys Thr Ser Phe
            20                  25                  30

Asn Pro Leu Pro Asn Phe Lys Thr Phe Pro Glu Arg Leu Val Ser Ala
        35                  40                  45

Ser Ile Ser Asn Val Val His Phe Thr Ile Ile Phe Leu Val Phe Val
    50                  55                  60

Phe Ser Ile Ser Ile Met Pro Val Pro Leu Leu Val Lys Ala Ile Leu
65                  70                  75                  80

Ile Ile Val Leu Ile Tyr Ile Asn Phe Ala Pro Ile Phe Ala Gln Phe
                85                  90                  95

Phe Asn Gly Gly Leu Pro Ile Leu Ser Trp Val Ile Leu Phe Phe Asn
            100                 105                 110

Ser Lys Asn Ile Pro Leu Ser Met Lys Arg Pro Ile Ser Val Lys Val
        115                 120                 125

Leu Pro Ala Leu Glu Thr Ile Phe Tyr Gly Asp Asn Leu Ser Glu Ile
    130                 135                 140

Leu Ala Ser His Thr Asn Thr Phe Leu Asp Leu Phe Ala Trp Phe Pro
145                 150                 155                 160

Tyr Gly Ile Val His Phe Ser Leu Pro Phe Val Val Ala Ala Leu Ile
                165                 170                 175

Phe Leu Phe Ala Pro Pro Lys Thr Leu Pro Gln Phe Cys Trp Ser Phe
            180                 185                 190

Gly Tyr Met Asn Leu Val Gly Val Ile Ile Gln Asn Leu Val Phe Ser
        195                 200                 205

Cys Ala Pro Pro Trp Tyr Lys Val Leu His Gly Leu Asp Lys Ala Asn
    210                 215                 220

Tyr Ser Met Lys Gly Ser Pro Gly Gly Leu Gly Arg Ile Asp Gly Leu
225                 230                 235                 240

Phe Gly Phe Asp Met Tyr Thr Ser Gly Phe Thr Asn Ser Pro Leu Ile
                245                 250                 255

Phe Gly Ala Met Pro Ser Leu His Ser Gly Cys Ser Thr Met Asp Ala
            260                 265                 270

Leu Trp Leu Ser Tyr Leu Phe Pro Lys Phe Thr Pro Leu Phe Val Phe
        275                 280                 285

Tyr Val Cys Trp Leu Trp Phe Ser Thr Met Tyr Leu Thr His His Tyr
    290                 295                 300

Phe Ile Asp Val Val Val Gly Ser Cys Leu Ala Ile Gly Phe Phe Thr
305                 310                 315                 320

Ala Val Lys Trp Arg Gly Arg Leu Pro Val Asn Asp Leu Phe Cys Arg
                325                 330                 335

Trp Asp His Asp Thr Leu Lys Phe His Asp Ile Trp Asn Glu Asp Pro
            340                 345                 350

Leu Arg Glu Asn Tyr Pro Leu Asp Leu Glu Ser Ser Lys Asp Leu Glu
        355                 360                 365

Asn Gln Ile Pro Leu Ser Asp Leu Asn Ser Ser Cys Asp Glu Gln Pro

370                    375                    380

Ser Ser Pro Lys Gly Phe Asn Arg Asn Gln Asn Asn Asn Leu Gly Asp
385                 390                 395                 400

Lys Gly Gln Gly Leu Glu Gln Val Asp Asp Gln Gly Glu Glu Tyr Asp
                405                 410                 415

Asn Tyr Asp Phe Asp Asp Ser Asp Asp Asp Gln Ile Ala Glu Ala Asn
            420                 425                 430

Asp Val Glu Lys Ser Ala Asn Lys Thr Ile Lys Lys Thr Pro Lys Ser
        435                 440                 445

Tyr Lys Glu Ser Phe Glu Ala Thr Leu Ser Thr Asn Leu Asn Ser Gln
    450                 455                 460

Ile Thr Asn Phe Pro Ser Ser Thr His Ile Pro Leu Asn Ser Ser Arg
465                 470                 475                 480

(2) INFORMATION FOR SEQ ID NO:6:

     (i) SEQUENCE CHARACTERISTICS:
          (A) LENGTH: 2000 base pairs
          (B) TYPE: nucleic acid
          (C) STRANDEDNESS: single
          (D) TOPOLOGY: linear

    (ii) MOLECULE TYPE: mRNA

   (iii) HYPOTHETICAL: NO

    (iv) ANTI-SENSE: NO

    (xi) SEQUENCE DESCRIPTION: SEQ ID NO:6:

ACAGUAAAAA GAAAUUGCGC UGCAUGAAAC UUUUUUUUUU UUUUUUUUCG GUGUUUCUUC      60

UCGUUUUUCU CGUUUUUCUC GUUUUUCUCG GUUUCUUUUG AGAAUGUAAU GAAAUUUGGA     120

AACUGUACAA ACAUUCUCAG UGUUGAUAUU AACAAUAAUA ACCACAUUGA CAUUGACAAC     180

AUCAUUAUUA GGUUCUUUCC AUUUGUAUUA UUUCCAUCUC GGACACGUCA GCUAUAGACC     240

GUCGACACUC CCUAUUCCCU GCACCAACUA GGAAGAACAG GAAGAACAAG AAGAACAGAA     300

UGCUCUCGCU CCCGGCCCUU AUUAGGCGCC UGUAUUCCUC CUUUAUUUCG GCAAGAGAAA     360

AGGGUAAUUC AUUGCCUGCA UUAAAGACAU CCUUUAACCC CCUCCCUAAC UUCAAGACCU     420

UCCCGGAGAG ACUGGUCUCG GCAUCCAUAU CCAAUGUGGU CCAUUUCACG AUUAUCUUUC     480

UUGUCUUUGU CUUCUCCAUC UCAAUAAUGC CUGUACCCCU ACUUGUGAAA GCCAUUCUUA     540

UCAUUGUCUU GAUUUACAUC AACUUUGCUC CAAUUUUCGC CCAGUUCUUU AACGGCGGCU     600

UGCCGAUCCU CUCAUGGGUC AUUUUGUUCU UUAACUCAAA GAACAUACCU CUGUCAAUGA     660

AGAGACCCAU CUCCGUCAAG GUCUUGCCCG CAUUGGAGAC AAUCUUCUAU GGCGAUAAUU     720

UAUCUGAAAU CUUGGCUUCU CAUACAAACA CUUUCUUGGA CUUGUUUGCU UGGUUCCCAU     780

AUGGUAUAGU CCAUUUCUCC CUGCCCUUUG UUGUGGCAGC CCUUAUUUUU UUAUUUGCCC 840

CCCCUAAAAC CUUACCCCAA UUUUGUUGGU CCUUUGGUUA UAUGAAUUUG GUUGGCGUCA 900

UUAUCCAAAA CUUGGUUUUC UCAUGUGCUC CACCAUGGUA UAAAGUGCUG CAUGGGUUGG 960

ACAAGGCAAA CUAUUCAAUG AAGGGAUCGC CAGGAGGAUU GGGUAGAAUC GAUGGACUGU 1020

UUGGUUUCGA UAUGUACACC UCGGGUUUCA CCAAUUCUCC CUUGAUUUUC GGUGCAAUGC 1080

CCUCCUUGCA UUCGGGAUGU UCAACUAUGG ACGCAUUAUG GUUGUCUUAU CUAUUCCCUA 1140

AAUUCACCCC UCUAUUUGUU UUCUACGUUU GCUGGUUAUG GUUCUCUACA AUGUAUUUGA 1200

CUCAUCAUUA CUUCAUCGAU GUUGUUGUUG GUUCUUGUUU GGCAAUUGGA UUCUUCACUG 1260

CAGUCAAAUG GAGAGGCAGA UUACCCGUAA AUGAUCUAUU UUGUAGGUGG GACCAUGAUA 1320

CCCUGAAAUU CCAUGACAUU UGGAACGAGG ACCCACUGAG GGAGAACUAU CCCUUGGACU 1380

UGGAGUCUUC AAAAGACUUG GAAAAUCAGA UACCUUUGAG UGAUUUGAAU UCAUCAUGUG 1440

AUGAACAACC AAGUUCACCA AAGGGAUUUA AUAGGAACCA GAAUAAUAAC UUGGGUGAUA 1500

AGGGACAGGG AUUAGAACAA GUAGACGAUC AAGGAGAAGA AUAUGACAAU UACGAUUUUG 1560

AUGAUUCUGA UGACGACCAA AUUGCUGAAG CUAAUGAUGU GGAGAAAUCG GCUAACAAAA 1620

CAAUAAAGAA GACUCCAAAG UCAUACAAGG AAUCAUUCGA AGCAACAUUA UCUACAAACU 1680

UGAACUCACA AAUUACAAAU UUCCCAUCUU CCACUCAUAU ACCUCUUAAU UCCAGUCGUU 1740

GACACAAACU CGGUUCUUUA AUAACUACCA CUUCGCCCUU UACUUAGCGU CUUUGAAAUU 1800

AUAGAUCUGG AAUUAGAAAU AAACUACCUU UUUUUUUGGC CAAUUGGAAA AAUUUAAUAC 1860

AACUCUUAUC AAGUCACUCC UUUAUUUUCA AUCUUUUUCU UUAUGUACAU GUAAUUUUAC 1920

AGAGUUCAAU AGUUAAUUAU UUAAUUACUU AUUUUCACUU UAUAGUUACA CUCUUCUCCC 1980

CACGUCCUUU UUUCUUAUCA 2000

(2) INFORMATION FOR SEQ ID NO:7:

(i) SEQUENCE CHARACTERISTICS:
(A) LENGTH: 1949 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(ix) FEATURE:
(A) NAME/KEY: CDS
(B) LOCATION: 162..1680

(xi) SEQUENCE DESCRIPTION: SEQ ID NO:7:

CACACACACA CACGGGTTAG AGCATCTGAA CTTCACGACA ACTGTTTTCT GTACAACCAA 60

AACAAACCAC AACACAAGAC AACACAACAC AGTACTTTGC TGACATTCAA TACCAATACC 120

CGTCTTTTTT TTTTCACCAC ACAACGATAT TGCTTTGAAG G ATG TCA CGC ACT 173
Met Ser Arg Thr
1

TAC AAT GGG TCT TCC TCC TCA TCA ATA TTT CTG AAA CCA CGT CGA TTT 221
Tyr Asn Gly Ser Ser Ser Ser Ser Ile Phe Leu Lys Pro Arg Arg Phe
5                   10                  15                  20

TTC AAT AAC TAC TTT CTA CTG GAA AAA TCA CCA ACT TCA ACA TTA CCT 269
Phe Asn Asn Tyr Phe Leu Leu Glu Lys Ser Pro Thr Ser Thr Leu Pro
                25                  30                  35

GAT TTA GAA ACA AAC ACT AGC ATC AGA ACT AGT CTT CAT AGA CTA AGA 317
Asp Leu Glu Thr Asn Thr Ser Ile Arg Thr Ser Leu His Arg Leu Arg
            40                  45                  50

CAT CAC AAA TTC ATA AAG GGA GAG ATT TTA CAC TAT GGG TTT CTT TCT 365
His His Lys Phe Ile Lys Gly Glu Ile Leu His Tyr Gly Phe Leu Ser
        55                  60                  65

TTT GTT TGG TTA TTT GTC TTT ACC ATA TAC CCA GCA TCC ATC TTG TTT 413
Phe Val Trp Leu Phe Val Phe Thr Ile Tyr Pro Ala Ser Ile Leu Phe
    70                  75                  80

AAA ATT CCA GTT TTA CTT GCA TTG CTA ACC ATT ATT ACT ATT CCA GCA 461
Lys Ile Pro Val Leu Leu Ala Leu Leu Thr Ile Ile Thr Ile Pro Ala
85                  90                  95                  100

ACT TCA CAA TTT TTT TTA CCA GCA CTT CCC ATT TTG ACT TGG TTG GGT 509
Thr Ser Gln Phe Phe Leu Pro Ala Leu Pro Ile Leu Thr Trp Leu Gly
                105                 110                 115

TTT TAC TTC ACT AGT TCC AAA ATA CCT CAT TCT TGG AAA CCA GCT ATT 557
Phe Tyr Phe Thr Ser Ser Lys Ile Pro His Ser Trp Lys Pro Ala Ile
            120                 125                 130

TCA GTC AAG GTT TTA CCA GCC ATG GAG ACT ATC TTG TAT GGT GAT GAT 605
Ser Val Lys Val Leu Pro Ala Met Glu Thr Ile Leu Tyr Gly Asp Asp
        135                 140                 145

TTA TCA AAT GTA TTG GCA ACC ATC ACT AAC CGT GGT TTG GAT ATT TTG 653
Leu Ser Asn Val Leu Ala Thr Ile Thr Asn Arg Gly Leu Asp Ile Leu
    150                 155                 160

GGC TGG TTA CCT TAT GGT TTA TTT CAT TTT GGT GCT CCA TTT GTT GTA 701
Gly Trp Leu Pro Tyr Gly Leu Phe His Phe Gly Ala Pro Phe Val Val
165                 170                 175                 180

GCT GCA ATT ATG TTT TTC TTT GCT CCA CCA ACT AGT TTA AGA TCA TTT 749
Ala Ala Ile Met Phe Phe Phe Ala Pro Pro Thr Ser Leu Arg Ser Phe
                185                 190                 195

GCG TTT GCT TTT GGT TAT ATG AAC TTG TTT GGG GTT ATT ATT CAA ATG 797
Ala Phe Ala Phe Gly Tyr Met Asn Leu Phe Gly Val Ile Ile Gln Met
            200                 205                 210

ATG TTT CCA GCT GCT CCA CCT TGG TAC AAG AAT TTG TAT GGA TTA CAA 845
Met Phe Pro Ala Ala Pro Pro Trp Tyr Lys Asn Leu Tyr Gly Leu Gln
        215                 220                 225

CCA GCC AAC TAT ACC ATG CAT GGC TCA CCA GGT GGA TTG GGC CGT ATA 893

Pro Ala Asn Tyr Thr Met His Gly Ser Pro Gly Gly Leu Gly Arg Ile
    230                 235                 240

GAT GAA TTA TTG GGA GTC GAT ATG TAC ACC ACT GGG TTC TCC AAC TCA 941
Asp Glu Leu Leu Gly Val Asp Met Tyr Thr Thr Gly Phe Ser Asn Ser
245                 250                 255                 260

CCG GTT ATT TTT GGT GCT TTC CCA TCA TTA CAT TCA GGT TGT TGT ATT 989
Pro Val Ile Phe Gly Ala Phe Pro Ser Leu His Ser Gly Cys Cys Ile
                265                 270                 275

ATG GAG GTT TTG TTT ATG TGT TGG TTA TTT CCC CGT TTG AAA GTA CTT 1037
Met Glu Val Leu Phe Met Cys Trp Leu Phe Pro Arg Leu Lys Val Leu
            280                 285                 290

TGG GTG TTT TAT GCA TCT TGG TTA TGG TGG AGT ACA ATG TAC TTG ACT 1085
Trp Val Phe Tyr Ala Ser Trp Leu Trp Trp Ser Thr Met Tyr Leu Thr
        295                 300                 305

CAT CAC TAT TTT GTT GAT TTA ACT GGT GGT GCT GTA TTA TCA TTG GTT 1133
His His Tyr Phe Val Asp Leu Thr Gly Gly Ala Val Leu Ser Leu Val
    310                 315                 320

GTT TTT GAA TTT GTC AAG TTT AAA TAC CTA CCT AAG GCT AAC AAG CGT 1181
Val Phe Glu Phe Val Lys Phe Lys Tyr Leu Pro Lys Ala Asn Lys Arg
325                 330                 335                 340

TGT CGT TGG TCT TAT ACG GAA TTG GAA TAC TAC AGC ACA ACC AAT GAT 1229
Cys Arg Trp Ser Tyr Thr Glu Leu Glu Tyr Tyr Ser Thr Thr Asn Asp
                345                 350                 355

GAC CCT TTA GGA GGT AAC AGT GCT GCC ACT CGA GTT GTT CGC GGC GGC 1277
Asp Pro Leu Gly Gly Asn Ser Ala Ala Thr Arg Val Val Arg Gly Gly
            360                 365                 370

GAT TTG GAA AAT TTT GCA TAT TCA AGA GTA TAC CCA TCA TTG TCA CAG 1325
Asp Leu Glu Asn Phe Ala Tyr Ser Arg Val Tyr Pro Ser Leu Ser Gln
        375                 380                 385

CAG CAA CAA TTG CAA GAG CAT CCG CAA CAA CAA CAA CAA TCA CTG TCT 1373
Gln Gln Gln Leu Gln Glu His Pro Gln Gln Gln Gln Gln Ser Leu Ser
    390                 395                 400

AAT CAA GAA AAC ATA CAA GAG GAG GTG TTT GAA ATG GGC ACC ATC TCA 1421
Asn Gln Glu Asn Ile Gln Glu Glu Val Phe Glu Met Gly Thr Ile Ser
405                 410                 415                 420

CGT TCA AGA TCA TCA CCA AGG TCC TTT GTT TCT CTT GGA AGT AGC ACC 1469
Arg Ser Arg Ser Ser Pro Arg Ser Phe Val Ser Leu Gly Ser Ser Thr
                425                 430                 435

AAT GTT GCT GAA GAG AAC TTT GAC CTT GCT GAT GAG GAA GAA GAT ATC 1517
Asn Val Ala Glu Glu Asn Phe Asp Leu Ala Asp Glu Glu Glu Asp Ile
            440                 445                 450

GGT TCA GGA TCA TCA AAA ACT CCC TCA GTA TTT GAA GAA GAT ACA CAA 1565
Gly Ser Gly Ser Ser Lys Thr Pro Ser Val Phe Glu Glu Asp Thr Gln
        455                 460                 465

AAT TAC GTC GTT TCT CAA ACT ACC TCC GTT GAA GAC TTG ACT AAT AAT 1613
Asn Tyr Val Val Ser Gln Thr Thr Ser Val Glu Asp Leu Thr Asn Asn
    470                 475                 480

ACA CAA GCA TCA GCT GCA GGC AAC GGA GCT ACC GAT AAG TCA TCA AGA 1661
Thr Gln Ala Ser Ala Ala Gly Asn Gly Ala Thr Asp Lys Ser Ser Arg

485                490                495                500

CTT GGA ATC AAT GGT TGA A CAAAGGGTAG ATGTATTAGA AGAGTAATCG          1710
Leu Gly Ile Asn Gly  *
                505

TTTCTTTTTT AATGGCTGCA TTTGTCTAGA AGAGTTCGAT TATAACGATG TTTGATGATG    1770

TTTATGTTGA TGATTCACGA TTTTTACGAA TTGTACAAGA ACCCTATTCA CAACCTGAGA    1830

TGGGATTAGA ATGTATTGAA CTTGGAAAAG ATATGGGACG TGTGCTTGGA ATGTCCTCCT    1890

TTTCTCATAT CCACTGCCCC ATTGCTATCC CTTTACAAGA TTATTACCAT GTTATGTCT     1949

(2) INFORMATION FOR SEQ ID NO:8:

        (i) SEQUENCE CHARACTERISTICS:
            (A) LENGTH: 506 amino acids
            (B) TYPE: amino acid
            (D) TOPOLOGY: linear

       (ii) MOLECULE TYPE: protein

       (xi) SEQUENCE DESCRIPTION: SEQ ID NO:8:

Met Ser Arg Thr Tyr Asn Gly Ser Ser Ser Ser Ser Ile Phe Leu Lys
  1               5                  10                  15

Pro Arg Arg Phe Phe Asn Asn Tyr Phe Leu Leu Glu Lys Ser Pro Thr
             20                  25                  30

Ser Thr Leu Pro Asp Leu Glu Thr Asn Thr Ser Ile Arg Thr Ser Leu
         35                  40                  45

His Arg Leu Arg His His Lys Phe Ile Lys Gly Glu Ile Leu His Tyr
     50                  55                  60

Gly Phe Leu Ser Phe Val Trp Leu Phe Val Phe Thr Ile Tyr Pro Ala
 65                  70                  75                  80

Ser Ile Leu Phe Lys Ile Pro Val Leu Leu Ala Leu Leu Thr Ile Ile
                 85                  90                  95

Thr Ile Pro Ala Thr Ser Gln Phe Phe Leu Pro Ala Leu Pro Ile Leu
            100                 105                 110

Thr Trp Leu Gly Phe Tyr Phe Thr Ser Ser Lys Ile Pro His Ser Trp
        115                 120                 125

Lys Pro Ala Ile Ser Val Lys Val Leu Pro Ala Met Glu Thr Ile Leu
    130                 135                 140

Tyr Gly Asp Asp Leu Ser Asn Val Leu Ala Thr Ile Thr Asn Arg Gly
145                 150                 155                 160

Leu Asp Ile Leu Gly Trp Leu Pro Tyr Gly Leu Phe His Phe Gly Ala
                165                 170                 175

Pro Phe Val Val Ala Ala Ile Met Phe Phe Phe Ala Pro Pro Thr Ser
            180                 185                 190

Leu Arg Ser Phe Ala Phe Ala Phe Gly Tyr Met Asn Leu Phe Gly Val
        195                 200                 205

Ile Ile Gln Met Met Phe Pro Ala Ala Pro Pro Trp Tyr Lys Asn Leu
    210                 215                 220

Tyr Gly Leu Gln Pro Ala Asn Tyr Thr Met His Gly Ser Pro Gly Gly
225                 230                 235                 240

Leu Gly Arg Ile Asp Glu Leu Leu Gly Val Asp Met Tyr Thr Thr Gly
                245                 250                 255

Phe Ser Asn Ser Pro Val Ile Phe Gly Ala Phe Pro Ser Leu His Ser
            260                 265                 270

Gly Cys Cys Ile Met Glu Val Leu Phe Met Cys Trp Leu Phe Pro Arg
        275                 280                 285

Leu Lys Val Leu Trp Val Phe Tyr Ala Ser Trp Leu Trp Trp Ser Thr
    290                 295                 300

Met Tyr Leu Thr His His Tyr Phe Val Asp Leu Thr Gly Gly Ala Val
305                 310                 315                 320

Leu Ser Leu Val Val Phe Glu Phe Val Lys Phe Lys Tyr Leu Pro Lys
                325                 330                 335

Ala Asn Lys Arg Cys Arg Trp Ser Tyr Thr Glu Leu Glu Tyr Tyr Ser
            340                 345                 350

Thr Thr Asn Asp Asp Pro Leu Gly Gly Asn Ser Ala Ala Thr Arg Val
        355                 360                 365

Val Arg Gly Gly Asp Leu Glu Asn Phe Ala Tyr Ser Arg Val Tyr Pro
    370                 375                 380

Ser Leu Ser Gln Gln Gln Gln Leu Gln Glu His Pro Gln Gln Gln Gln
385                 390                 395                 400

Gln Ser Leu Ser Asn Gln Glu Asn Ile Gln Glu Glu Val Phe Glu Met
                405                 410                 415

Gly Thr Ile Ser Arg Ser Arg Ser Ser Pro Arg Ser Phe Val Ser Leu
            420                 425                 430

Gly Ser Ser Thr Asn Val Ala Glu Glu Asn Phe Asp Leu Ala Asp Glu
        435                 440                 445

Glu Glu Asp Ile Gly Ser Gly Ser Ser Lys Thr Pro Ser Val Phe Glu
    450                 455                 460

Glu Asp Thr Gln Asn Tyr Val Val Ser Gln Thr Thr Ser Val Glu Asp
465                 470                 475                 480

Leu Thr Asn Asn Thr Gln Ala Ser Ala Ala Gly Asn Gly Ala Thr Asp
                485                 490                 495

Lys Ser Ser Arg Leu Gly Ile Asn Gly  *
            500                 505

(2) INFORMATION FOR SEQ ID NO:9:

     (i) SEQUENCE CHARACTERISTICS:
          (A) LENGTH: 1788 base pairs
          (B) TYPE: nucleic acid
          (C) STRANDEDNESS: single
          (D) TOPOLOGY: linear

(ii) MOLECULE TYPE: RNA (genomic)

(iii) HYPOTHETICAL: NO

(iv) ANTI-SENSE: NO

(xi) SEQUENCE DESCRIPTION: SEQ ID NO:9:

AUGUCACGCA CUUACAAUGG GUCUUCCUCC UCAUCAAUAU UUCUGAAACC ACGUCGAUUU 60

UUCAAUAACU ACUUUCUACU GGAAAAAUCA CCAACUUCAA CAUUACCUGA UUUAGAAACA 120

AACACUAGCA UCAGAACUAG UCUUCAUAGA CUAAGACAUC ACAAAUUCAU AAAGGGAGAG 180

AUUUUACACU AUGGGUUUCU UUCUUUUGUU UGGUUAUUUG UCUUUACCAU AUACCCAGCA 240

UCCAUCUUGU UUAAAAUUCC AGUUUUACUU GCAUUGCUAA CCAUUAUUAC UAUUCCAGCA 300

ACUUCACAAU UUUUUUUACC AGCACUUCCC AUUUUGACUU GGUUGGGUUU UUACUUCACU 360

AGUUCCAAAA UACCUCAUUC UUGGAAACCA GCUAUUUCAG UCAAGGUUUU ACCAGCCAUG 420

GAGACUAUCU UGUAUGGUGA UGAUUUAUCA AAUGUAUUGG CAACCAUCAC UAACCGUGGU 480

UUGGAUAUUU UGGGCUGGUU ACCUUAUGGU UUAUUUCAUU UUGGUGCUCC AUUUGUUGUA 540

GCUGCAAUUA UGUUUUUCUU UGCUCCACCA ACUAGUUUAA GAUCAUUUGC GUUUGCUUUU 600

GGUUAUAUGA ACUUGUUUGG GGUUAUUAUU CAAAUGAUGU UUCCAGCUGC UCCACCUUGG 660

UACAAGAAUU UGUAUGGAUU ACAACCAGCC AACUAUACCA UGCAUGGCUC ACCAGGUGGA 720

UUGGGCCGUA UAGAUGAAUU AUUGGGAGUC GAUAUGUACA CCACUGGGUU CUCCAACUCA 780

CCGGUUAUUU UUGGUGCUUU CCCAUCAUUA CAUUCAGGUU GUUGUAUUAU GGAGGUUUUG 840

UUUAUGUGUU GGUUAUUUCC CCGUUUGAAA GUACUUUGGG UGUUUUAUGC AUCUUGGUUA 900

UGGUGGAGUA CAAUGUACUU GACUCAUCAC UAUUUUGUUG AUUUAACUGG UGGUGCUGUA 960

UUAUCAUUGG UUGUUUUUGA AUUUGUCAAG UUUAAAUACC UACCUAAGGC UAACAAGCGU 1020

UGUCGUUGGU CUUAUACGGA AUUGGAAUAC UACAGCACAA CCAAUGAUGA CCCUUUAGGA 1080

GGUAACAGUG CUGCCACUCG AGUUGUUCGC GGCGGCGAUU UGGAAAAUUU UGCAUAUUCA 1140

AGAGUAUACC CAUCAUUGUC ACAGCAGCAA CAAUUGCAAG AGCAUCCGCA ACAACAACAA 1200

CAAUCACUGU CUAAUCAAGA AAACAUACAA GAGGAGGUGU UUGAAAUGGG CACCAUCUCA 1260

CGUUCAAGAU CAUCACCAAG GUCCUUUGUU UCUCUUGGAA GUAGCACCAA UGUUGCUGAA 1320

GAGAACUUUG ACCUUGCUGA UGAGGAAGAA GAUAUCGGUU CAGGAUCAUC AAAAACUCCC 1380

UCAGUAUUUG AAGAAGAUAC ACAAAAUUAC GUCGUUUCUC AAACUACCUC CGUUGAAGAC 1440

UUGACUAAUA AUACACAAGC AUCAGCUGCA GGCAACGGAG CUACCGAUAA GUCAUCAAGA 1500

CUUGGAAUCA AUGGUUGAAC AAAGGGUAGA UGUAUUAGAA GAGUAAUCGU UUCUUUUUUA 1560

AUGGCUGCAU UUGUCUAGAA GAGUUCGAUU AUAACGAUGU UUGAUGAUGU UUAUGUUGAU 1620

GAUUCACGAU UUUUACGAAU UGUACAAGAA CCCUAUUCAC AACCUGAGAU GGGAUUAGAA 1680

UGUAUUGAAC UUGGAAAAGA UAUGGGACGU GUGCUUGGAA UGUCCUCCUU UUCUCAUAUC 1740

CACUGCCCCA UUGCUAUCCC UUUACAAGAU UAUUACCAUG UUAUGUCU 1788

(2) INFORMATION FOR SEQ ID NO:10:

(i) SEQUENCE CHARACTERISTICS:
(A) LENGTH: 2214 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(ix) FEATURE:
(A) NAME/KEY: CDS
(B) LOCATION: 601..1974

(xi) SEQUENCE DESCRIPTION: SEQ ID NO:10:

CGATGGTGAT TTCTTTTTCT CTCCTATTCC TGTACCCCGC AAATGTGTGA TTTCAATCCC 60

AAGGTGCCAA AGCACACTGA TGTTGGCTTG AAAGACTCAG ATATGGAGCT TTGTAATATA 120

AGAAAAATGC CAAAGTGCTT TTCTTGCCAA TCATTGTCGA TTTGGACTTT TCTGCCTCTG 180

TCTCACTTTG CATCTTTAGT GACGCATACG GGAAAAAATG TAAAATGCGC TGACCATAAA 240

TGACATTCGG AACATAATGC ATAGAATATT AATACCGCAT ACCGATAACA GAAGTCACAA 300

ACGTCACAAA ACGTTTCAAA AAAACAGAAA CAGGCAAGCC TGAAAGTTGA ACACAGCCAC 360

TTCTTCCCCA AACTATGAAA ACTTGGTCGT TGCAAGATCA TTCACTTGCG CCCCTATTTT 420

ATAGCATGGA AAGGATGCGT TTCTGTTCCG GCCCATTTGC GCGCATTTGA GGTCACGTGC 480

GCTGCATCGT CACGGCCCAT TCCTTTTTCT ATCCTTTTAT TTTATGTTGC TTCAGCCCCA 540

ACGCTGAAAG GCCGTCTTTT CCAGCAGAAT CCATTACTCT GCTATATTTC CCTCATTCCA 600

ATG TCA ACG GTC CTA AGG TTC AAG CCT TTC CGC GTT GCT TAC AAG CAA 648
Met Ser Thr Val Leu Arg Phe Lys Pro Phe Arg Val Ala Tyr Lys Gln
1               5                   10                  15

TTC CAC AAT TAT TTT TTG CTG GAG AAA GAA GCA GGA TGC ACT TTG GCG 696
Phe His Asn Tyr Phe Leu Leu Glu Lys Glu Ala Gly Cys Thr Leu Ala
            20                  25                  30

GAT CTT GAT TTC GAT ACT GAT ATC CGC AAG TCC ATC CAG AAA CTA CGT 744
Asp Leu Asp Phe Asp Thr Asp Ile Arg Lys Ser Ile Gln Lys Leu Arg
        35                  40                  45

TCC CAT GAG TTT ACG CCG GGC GAA ATA GTG CAC TAC ACT TTC CTC ACG 792
Ser His Glu Phe Thr Pro Gly Glu Ile Val His Tyr Thr Phe Leu Thr
    50                  55                  60

CTG TTG ATC TTG TTT GTT TAT GTC ATT TTC CCC GCG TCG TTT TTG CTC 840

Leu Leu Ile Leu Phe Val Tyr Val Ile Phe Pro Ala Ser Phe Leu Leu
65                  70                  75                  80

AAA ACG CCC ATC TTG GCG GCT TTC GGC TTT TGT TTC TTA TTG CCC TTG      888
Lys Thr Pro Ile Leu Ala Ala Phe Gly Phe Cys Phe Leu Leu Pro Leu
                85                  90                  95

ACG TCC CAG TTT TTC GTG TCA GCG TTG CCC ATT TTC ACA TGG TTG GCG      936
Thr Ser Gln Phe Phe Val Ser Ala Leu Pro Ile Phe Thr Trp Leu Ala
            100                 105                 110

CTT TTC TTC TCT GCC AGC AAG ATC CCT GTG GAC TGG AAG CCT GTC ATT      984
Leu Phe Phe Ser Ala Ser Lys Ile Pro Val Asp Trp Lys Pro Val Ile
        115                 120                 125

AGC GTC AAG TTT CTT CCT GCC ATG GAA ACT ATC TTG TAC GGA GAC AAC     1032
Ser Val Lys Phe Leu Pro Ala Met Glu Thr Ile Leu Tyr Gly Asp Asn
    130                 135                 140

TTG TCG AAT GTT TTG GCA GCC ACC AAC AAC TGG GTG CTA GAT ATC TTG     1080
Leu Ser Asn Val Leu Ala Ala Thr Asn Asn Trp Val Leu Asp Ile Leu
145                 150                 155                 160

GCG TGG TTG CCC TAC GGT ATC ATC CAC TTT CTG TCT CCA TTT GTA GTT     1128
Ala Trp Leu Pro Tyr Gly Ile Ile His Phe Leu Ser Pro Phe Val Val
                165                 170                 175

GCG CTC TTC ATT TTC CTT TTT GCT CCG CCC ACT TCT TTG CGC TCG TTT     1176
Ala Leu Phe Ile Phe Leu Phe Ala Pro Pro Thr Ser Leu Arg Ser Phe
            180                 185                 190

GGC TTC GCC TTT GGC TAC ATG AAT GTG GTT GGT GTG TTG ATC CAG CTT     1224
Gly Phe Ala Phe Gly Tyr Met Asn Val Val Gly Val Leu Ile Gln Leu
        195                 200                 205

TGT TTC CCC TCA GCT GCT CCG TGG TAC AAG AAC TTG CAC GGC CTA GAA     1272
Cys Phe Pro Ser Ala Ala Pro Trp Tyr Lys Asn Leu His Gly Leu Glu
    210                 215                 220

CCT GCA AAT TAT TCC ATG AAC GGG TCT CCT GGT GGC TTG GGC CGC ATT     1320
Pro Ala Asn Tyr Ser Met Asn Gly Ser Pro Gly Gly Leu Gly Arg Ile
225                 230                 235                 240

GAC GAG CTT TTC GGT GTC GAC ATG TAC ACC ACT ACT TTC ATG AAC TCA     1368
Asp Glu Leu Phe Gly Val Asp Met Tyr Thr Thr Thr Phe Met Asn Ser
                245                 250                 255

CCA TTG GTT TTC GGC GCG TTC CCC TCG CTC CAT TCG GGC TGC GCA GTC     1416
Pro Leu Val Phe Gly Ala Phe Pro Ser Leu His Ser Gly Cys Ala Val
            260                 265                 270

ATG GAT GTG CTC TTT TTG TGC TGG CTC TTC CCT CGT TTC ACC TAT GTT     1464
Met Asp Val Leu Phe Leu Cys Trp Leu Phe Pro Arg Phe Thr Tyr Val
        275                 280                 285

TGG TGG AGC TAC GCC TGT CTT TTG TGG TGG AGC ACC ATG TAT TTG ACG     1512
Trp Trp Ser Tyr Ala Cys Leu Leu Trp Trp Ser Thr Met Tyr Leu Thr
    290                 295                 300

CAC CAC TAT TTT ATC GAC TTG ATC TTC GGT GCC GCT TTG TCT GTG CTA     1560
His His Tyr Phe Ile Asp Leu Ile Phe Gly Ala Ala Leu Ser Val Leu
305                 310                 315                 320

GTT TTC AAC TAT GTC AAG TAC ACG CAG TTG CCA GTG GTG GAC CCC ACC     1608
Val Phe Asn Tyr Val Lys Tyr Thr Gln Leu Pro Val Val Asp Pro Thr

                325                 330                 335

AAG TTC TGT CGC TGG CTG TAC ACC ACC GTG GAG CTT CTT GAC ATT AAC 1656
Lys Phe Cys Arg Trp Leu Tyr Thr Thr Val Glu Leu Leu Asp Ile Asn
            340                 345                 350

GCC AAC GAC CCA TTG CAG AGT TTC GTG CCA TTA CAC GGA GAC ATG GAG 1704
Ala Asn Asp Pro Leu Gln Ser Phe Val Pro Leu His Gly Asp Met Glu
        355                 360                 365

GAA GGT ATG CTG CAT CCC TTT TAC CAC GCG CCA GCG CTT GAG AAT TTC 1752
Glu Gly Met Leu His Pro Phe Tyr His Ala Pro Ala Leu Glu Asn Phe
    370                 375                 380

GAA ATG TCT ACT ATA AAC CGT TCA CGG GAA AGC GTG GCT GCG CCG TTT 1800
Glu Met Ser Thr Ile Asn Arg Ser Arg Glu Ser Val Ala Ala Pro Phe
385                 390                 395                 400

CCG ACC ATA GCC GAG GAT GTG GCT GAG GAA ACC GAC AGC AGC GCG TCC 1848
Pro Thr Ile Ala Glu Asp Val Ala Glu Glu Thr Asp Ser Ser Ala Ser
                405                 410                 415

AAT TCG GTT TTT GAT GCT GAG CGT TTT GAC GAA GAA ACG CAT CCT AGC 1896
Asn Ser Val Phe Asp Ala Glu Arg Phe Asp Glu Glu Thr His Pro Ser
            420                 425                 430

CTG CGT ACG TCC AGC ACC TCG TTG AAC GAC TTG CTT CTT CCC CTG AGC 1944
Leu Arg Thr Ser Ser Thr Ser Leu Asn Asp Leu Leu Leu Pro Leu Ser
        435                 440                 445

TCG AGC AGC TCA AAG CCC AAA AGA AGC ACG TAGCCTTTTA CATTGTCGCA 1994
Ser Ser Ser Ser Lys Pro Lys Arg Ser Thr
    450                 455

CCGCTCTTTG GCCAGGAGCA CTTTAATATT GGCCGTGTAT ACTACATAAA TGCGACGTCT 2054

ATCGTGATGC TATGTACATT GTGAGTCCTA GCTCGGTATA TGATATATCC TCTTTCGTTT 2114

TCGTTCGTAG CTTCCTTCAC TTCCTAATCG TTCCTTCTCC TTAGTTTGAC ACCTTCTCGA 2174

ACACTCTTTG GTAAGTAGTG CCCACATTCA AGTTGCCCTT 2214

(2) INFORMATION FOR SEQ ID NO:11:

(i) SEQUENCE CHARACTERISTICS:
(A) LENGTH: 458 amino acids
(B) TYPE: amino acid
(D) TOPOLOGY: linear

(ii) MOLECULE TYPE: protein

(xi) SEQUENCE DESCRIPTION: SEQ ID NO:11:

Met Ser Thr Val Leu Arg Phe Lys Pro Phe Arg Val Ala Tyr Lys Gln
1               5                   10                  15

Phe His Asn Tyr Phe Leu Leu Glu Lys Glu Ala Gly Cys Thr Leu Ala
            20                  25                  30

Asp Leu Asp Phe Asp Thr Asp Ile Arg Lys Ser Ile Gln Lys Leu Arg
        35                  40                  45

Ser His Glu Phe Thr Pro Gly Glu Ile Val His Tyr Thr Phe Leu Thr
    50                  55                  60

Leu Leu Ile Leu Phe Val Tyr Val Ile Phe Pro Ala Ser Phe Leu Leu
65                  70                  75                  80

Lys Thr Pro Ile Leu Ala Ala Phe Gly Phe Cys Phe Leu Leu Pro Leu
                85                  90                  95

Thr Ser Gln Phe Phe Val Ser Ala Leu Pro Ile Phe Thr Trp Leu Ala
            100                 105                 110

Leu Phe Phe Ser Ala Ser Lys Ile Pro Val Asp Trp Lys Pro Val Ile
        115                 120                 125

Ser Val Lys Phe Leu Pro Ala Met Glu Thr Ile Leu Tyr Gly Asp Asn
    130                 135                 140

Leu Ser Asn Val Leu Ala Ala Thr Asn Asn Trp Val Leu Asp Ile Leu
145                 150                 155                 160

Ala Trp Leu Pro Tyr Gly Ile Ile His Phe Leu Ser Pro Phe Val Val
                165                 170                 175

Ala Leu Phe Ile Phe Leu Phe Ala Pro Pro Thr Ser Leu Arg Ser Phe
            180                 185                 190

Gly Phe Ala Phe Gly Tyr Met Asn Val Val Gly Val Leu Ile Gln Leu
        195                 200                 205

Cys Phe Pro Ser Ala Ala Pro Trp Tyr Lys Asn Leu His Gly Leu Glu
    210                 215                 220

Pro Ala Asn Tyr Ser Met Asn Gly Ser Pro Gly Gly Leu Gly Arg Ile
225                 230                 235                 240

Asp Glu Leu Phe Gly Val Asp Met Tyr Thr Thr Thr Phe Met Asn Ser
                245                 250                 255

Pro Leu Val Phe Gly Ala Phe Pro Ser Leu His Ser Gly Cys Ala Val
            260                 265                 270

Met Asp Val Leu Phe Leu Cys Trp Leu Phe Pro Arg Phe Thr Tyr Val
        275                 280                 285

Trp Trp Ser Tyr Ala Cys Leu Leu Trp Trp Ser Thr Met Tyr Leu Thr
    290                 295                 300

His His Tyr Phe Ile Asp Leu Ile Phe Gly Ala Ala Leu Ser Val Leu
305                 310                 315                 320

Val Phe Asn Tyr Val Lys Tyr Thr Gln Leu Pro Val Val Asp Pro Thr
                325                 330                 335

Lys Phe Cys Arg Trp Leu Tyr Thr Thr Val Glu Leu Leu Asp Ile Asn
            340                 345                 350

Ala Asn Asp Pro Leu Gln Ser Phe Val Pro Leu His Gly Asp Met Glu
        355                 360                 365

Glu Gly Met Leu His Pro Phe Tyr His Ala Pro Ala Leu Glu Asn Phe
    370                 375                 380

Glu Met Ser Thr Ile Asn Arg Ser Arg Glu Ser Val Ala Ala Pro Phe
385                 390                 395                 400

Pro Thr Ile Ala Glu Asp Val Ala Glu Glu Thr Asp Ser Ser Ala Ser

                405                 410                 415

Asn Ser Val Phe Asp Ala Glu Arg Phe Asp Glu Glu Thr His Pro Ser
            420                 425                 430

Leu Arg Thr Ser Ser Thr Ser Leu Asn Asp Leu Leu Leu Pro Leu Ser
        435                 440                 445

Ser Ser Ser Ser Lys Pro Lys Arg Ser Thr
    450                 455

(2) INFORMATION FOR SEQ ID NO:12:

(i) SEQUENCE CHARACTERISTICS:
(A) LENGTH: 2214 base pairs
(B) TYPE: nucleic acid
(C) STRANDEDNESS: single
(D) TOPOLOGY: linear

(ii) MOLECULE TYPE: RNA (genomic)

(iii) HYPOTHETICAL: NO

(iv) ANTI-SENSE: NO

(xi) SEQUENCE DESCRIPTION: SEQ ID NO:12:

CGAUGGUGAU UUCUUUUUCU CUCCUAUUCC UGUACCCCGC AAAUGUGUGA UUUCAAUCCC 60

AAGGUGCCAA AGCACACUGA UGUUGGCUUG AAAGACUCAG AUAUGGAGCU UUGUAAUAUA 120

AGAAAAAUGC CAAAGUGCUU UUCUUGCCAA UCAUUGUCGA UUUGGACUUU UCUGCCUCUG 180

UCUCACUUUG CAUCUUUAGU GACGCAUACG GGAAAAAAUG UAAAAUGCGC UGACCAUAAA 240

UGACAUUCGG AACAUAAUGC AUAGAAUAUU AAUACCGCAU ACCGAUAACA GAAGUCACAA 300

ACGUCACAAA ACGUUUCAAA AAAACAGAAA CAGGCAAGCC UGAAAGUUGA ACACAGCCAC 360

UUCUUCCCCA AACUAUGAAA ACUUGGUCGU UGCAAGAUCA UUCACUUGCG CCCCUAUUUU 420

AUAGCAUGGA AAGGAUGCGU UUCUGUUCCG GCCCAUUUGC GCGCAUUUGA GGUCACGUGC 480

GCUGCAUCGU CACGGCCCAU UCCUUUUUCU AUCCUUUUAU UUUAUGUUGC UUCAGCCCCA 540

ACGCUGAAAG GCCGUCUUUU CCAGCAGAAU CCAUUACUCU GCUAUAUUUC CCUCAUUCCA 600

AUGUCAACGG UCCUAAGGUU CAAGCCUUUC CGCGUUGCUU ACAAGCAAUU CCACAAUUAU 660

UUUUUGCUGG AGAAAGAAGC AGGAUGCACU UUGGCGGAUC UUGAUUUCGA UACUGAUAUC 720

CGCAAGUCCA UCCAGAAACU ACGUUCCCAU GAGUUUACGC CGGGCGAAAU AGUGCACUAC 780

ACUUUCCUCA CGCUGUUGAU CUUGUUUGUU UAUGUCAUUU UCCCCGCGUC GUUUUUGCUC 840

AAAACGCCCA UCUUGGCGGC UUUCGGCUUU UGUUUCUUAU UGCCCUUGAC GUCCCAGUUU 900

UUCGUGUCAG CGUUGCCCAU UUUCACAUGG UUGGCGCUUU UCUUCUCUGC CAGCAAGAUC 960

CCUGUGGACU GGAAGCCUGU CAUUAGCGUC AAGUUUCUUC CUGCCAUGGA AACUAUCUUG 1020

UACGGAGACA ACUUGUCGAA UGUUUUGGCA GCCACCAACA ACUGGGUGCU AGAUAUCUUG 1080

GCGUGGUUGC CCUACGGUAU CAUCCACUUU CUGUCUCCAU UUGUAGUUGC GCUCUUCAUU 1140

UUCCUUUUUG CUCCGCCCAC UUCUUUGCGC UCGUUUGGCU UCGCCUUUGG CUACAUGAAU 1200

GUGGUUGGUG UGUUGAUCCA GCUUUGUUUC CCCUCAGCUG CUCCGUGGUA CAAGAACUUG 1260

CACGGCCUAG AACCUGCAAA UUAUUCCAUG AACGGGUCUC CUGGUGGCUU GGGCCGCAUU 1320

GACGAGCUUU UCGGUGUCGA CAUGUACACC ACUACUUUCA UGAACUCACC AUUGGUUUUC 1380

GGCGCGUUCC CCUCGCUCCA UUCGGGCUGC GCAGUCAUGG AUGUGCUCUU UUUGUGCUGG 1440

CUCUUCCCUC GUUUCACCUA UGUUUGGUGG AGCUACGCCU GUCUUUUGUG GUGGAGCACC 1500

AUGUAUUUGA CGCACCACUA UUUUAUCGAC UUGAUCUUCG GUGCCGCUUU GUCUGUGCUA 1560

GUUUUCAACU AUGUCAAGUA CACGCAGUUG CCAGUGGUGG ACCCCACCAA GUUCUGUCGC 1620

UGGCUGUACA CCACCGUGGA GCUUCUUGAC AUUAACGCCA ACGACCCAUU GCAGAGUUUC 1680

GUGCCAUUAC ACGGAGACAU GGAGGAAGGU AUGCUGCAUC CCUUUUACCA CGCGCCAGCG 1740

CUUGAGAAUU UCGAAAUGUC UACUAUAAAC CGUUCACGGG AAAGCGUGGC UGCGCCGUUU 1800

CCGACCAUAG CCGAGGAUGU GGCUGAGGAA ACCGACAGCA GCGCGUCCAA UUCGGUUUUU 1860

GAUGCUGAGC GUUUUGACGA AGAAACGCAU CCUAGCCUGC GUACGUCCAG CACCUCGUUG 1920

AACGACUUGC UUCUUCCCCU GAGCUCGAGC AGCUCAAAGC CCAAAAGAAG CACGUAGCCU 1980

UUUACAUUGU CGCACCGCUC UUUGGCCAGG AGCACUUUAA UAUUGGCCGU GUAUACUACA 2040

UAAAUGCGAC GUCUAUCGUG AUGCUAUGUA CAUUGUGAGU CCUAGCUCGG UAUAUGAUAU 2100

AUCCUCUUUC GUUUUCGUUC GUAGCUUCCU UCACUUCCUA AUCGUUCCUU CUCCUUAGUU 2160

UGACACCUUC UCGAACACUC UUUGGUAAGU AGUGCCCACA UUCAAGUUGC CCUU 2214

(2) INFORMATION FOR SEQ ID NO:13:

(i) SEQUENCE CHARACTERISTICS:
(A) LENGTH: 2935 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(ix) FEATURE:
(A) NAME/KEY: CDS
(B) LOCATION: 450..1757

(xi) SEQUENCE DESCRIPTION: SEQ ID NO:13:

ATTTTCTTCC CCATAACAAC TCTTCTCGCC CTTCCTCCGG CTCCGTGGCC AAATTGTTTT 60

ATGCAGCGCC TCCTAGCGAT TTAACCTCGT TCTCGTTGCC CTTGCCTGTC CGCCTTGCGT 120

CAGTACGACC CTTGCAACGT GACCTTCCCC AGAGTATCCT CGTTTGGCCG CTGGAGACCG 180

GAGCTTGCAC CCTCATAAAC TAGCTCTTCG AAATCAATTC TCCGTTCTCC AGAGATTATC 240

GGATCGAATC TCTCCGCTGT CGACACCTTT CGTCTCTCGG TGATCCTCGC CCTTGGAGTC 300

TCGTCACGTT GACGCCTTGA ACCCCTGGCC GCCAACTCCA CATAGGAGAC CACACTTCAT 360

TCTTCCCCCG CCATAATTGC AGCACCCTCC GTCTCCCTTC GAGCTCCTCC TGGATCATCA 420

AGTCCGAAAG GATTAGACTC GTCGCAGCG ATG AAT ACC ACC CTT CCA TCC TGG 473
Met Asn Thr Thr Leu Pro Ser Trp
1               5

AAG GAT CGG ACG CAA AAC CAG TTC GGC AAG CTC CAG ATC CAA GTC CCA 521
Lys Asp Arg Thr Gln Asn Gln Phe Gly Lys Leu Gln Ile Gln Val Pro
    10                  15                  20

TGG CGC ACC ATA CAA CTT CTC GTG CCG CAC CGT ATG CGA CGG AAG ATT 569
Trp Arg Thr Ile Gln Leu Leu Val Pro His Arg Met Arg Arg Lys Ile
25                  30                  35                  40

CGG TCC AAG CTG CGC AGT CGG ATC TCG CCT ACC TCA TCG ATA TCC TCG 617
Arg Ser Lys Leu Arg Ser Arg Ile Ser Pro Thr Ser Ser Ile Ser Ser
                45                  50                  55

TTG CAG ACG TCA TTC TCA CCT GTC GAT ACA CTC AGG TCG CTG CAA AGT 665
Leu Gln Thr Ser Phe Ser Pro Val Asp Thr Leu Arg Ser Leu Gln Ser
            60                  65                  70

CAT AGA TGG ACG CTC TAT GAC TTT CAG TAT CTT TTG CTG CTG ATT GTC 713
His Arg Trp Thr Leu Tyr Asp Phe Gln Tyr Leu Leu Leu Leu Ile Val
        75                  80                  85

GGC ATA TTC TCG CTG AGC GTT ATG GAA TCA CCT GGA CCA TTG GCA AAG 761
Gly Ile Phe Ser Leu Ser Val Met Glu Ser Pro Gly Pro Leu Ala Lys
    90                  95                  100

ACC GCC GCG TTT ACG CTA CTT CTC GTC TCT CTC CTT CTC CCG ATT ACG 809
Thr Ala Ala Phe Thr Leu Leu Leu Val Ser Leu Leu Leu Pro Ile Thr
105                 110                 115                 120

CGC CAG TTC TTC TTG CCA TTC CTC CCG ATT GCA GGA TGG CTT ATA TTT 857
Arg Gln Phe Phe Leu Pro Phe Leu Pro Ile Ala Gly Trp Leu Ile Phe
                125                 130                 135

TTC TAC GCT TGC CAG TTC ATC CCG AGC GAC TGG CGC CCT GCA ATC TGG 905
Phe Tyr Ala Cys Gln Phe Ile Pro Ser Asp Trp Arg Pro Ala Ile Trp
            140                 145                 150

GTT CGC GTG CTG CCG GCT CTG GAA AAC ATT CTC TAC GGT GCT AAT ATC 953
Val Arg Val Leu Pro Ala Leu Glu Asn Ile Leu Tyr Gly Ala Asn Ile
        155                 160                 165

AGT AAC ATC CTT TCC GCT CAC CAA AAT GTG GTG CTT GAC GTT TTG GCG 1001
Ser Asn Ile Leu Ser Ala His Gln Asn Val Val Leu Asp Val Leu Ala
    170                 175                 180

TGG CTT CCC TAC GGA ATC TGC CAT TAT GGC GCG CCA TTT GTG TGC TCA 1049
Trp Leu Pro Tyr Gly Ile Cys His Tyr Gly Ala Pro Phe Val Cys Ser
185                 190                 195                 200

GCG ATC ATG TTC ATC TTT GGT CCT CCC GGC ACC GTC CCC CTT TTC GCT 1097
Ala Ile Met Phe Ile Phe Gly Pro Pro Gly Thr Val Pro Leu Phe Ala
                205                 210                 215

CGA ACT TTT GGA TAC ATC AGC ATG GCT GCA GTC ACC ATT CAG CTG TTT 1145
Arg Thr Phe Gly Tyr Ile Ser Met Ala Ala Val Thr Ile Gln Leu Phe
            220                 225                 230

TTC CCC TGC TCT CCT CCG TGG TAC GAA AAT CTG TAT GGT TTG GCT CCG 1193
Phe Pro Cys Ser Pro Pro Trp Tyr Glu Asn Leu Tyr Gly Leu Ala Pro
        235                 240                 245

GCT GAT TAC TCC ATG CCG GGT AAT CCT GCG GGC CTT GCT CGC ATC GAT 1241
Ala Asp Tyr Ser Met Pro Gly Asn Pro Ala Gly Leu Ala Arg Ile Asp
    250                 255                 260

GAG CTT TTT GGG ATA GAC TTG TAC ACA TCG GGC TTC AGA CAA TCT CCC 1289
Glu Leu Phe Gly Ile Asp Leu Tyr Thr Ser Gly Phe Arg Gln Ser Pro
265                 270                 275                 280

GTC GTG TTT GGC GCA TTT CCT TCC CTA CAT GCC GCT GAT TCG ACA CTT 1337
Val Val Phe Gly Ala Phe Pro Ser Leu His Ala Ala Asp Ser Thr Leu
                285                 290                 295

GCA GCT CTA TTT ATG AGC CAA GTG TTC CCA CGG TTG AAG CCC TTG TTT 1385
Ala Ala Leu Phe Met Ser Gln Val Phe Pro Arg Leu Lys Pro Leu Phe
            300                 305                 310

GTC ATC TAT ACT CTC TGG ATG TGG TGG GCT ACA ATG TAT CTT TCG CAC 1433
Val Ile Tyr Thr Leu Trp Met Trp Trp Ala Thr Met Tyr Leu Ser His
        315                 320                 325

CAC TAC GCT GTT GAT CTG GTC GGT GGT GGC CTC TTG GCA ACT GTC GCG 1481
His Tyr Ala Val Asp Leu Val Gly Gly Gly Leu Leu Ala Thr Val Ala
    330                 335                 340

TTC TAC TTT GCT AAA ACG CGG TTC ATG CCT CGC GTC CAG AAT GAT AAG 1529
Phe Tyr Phe Ala Lys Thr Arg Phe Met Pro Arg Val Gln Asn Asp Lys
345                 350                 355                 360

ATG TTC CGC TGG GAC TAC GAT TAT GTT GAG TCA GGC GAT TCC GCA CTC 1577
Met Phe Arg Trp Asp Tyr Asp Tyr Val Glu Ser Gly Asp Ser Ala Leu
                365                 370                 375

GAC TAT GGG TAC GGT CCA GCC AGC TTC GAA GGC GAA TTC AAC CTT GAT 1625
Asp Tyr Gly Tyr Gly Pro Ala Ser Phe Glu Gly Glu Phe Asn Leu Asp
            380                 385                 390

AGC GAT GAG TGG ACC GTT GGT TCT TCG TCA TCC ATT TCG TCC GGC TCC 1673
Ser Asp Glu Trp Thr Val Gly Ser Ser Ser Ser Ile Ser Ser Gly Ser
        395                 400                 405

CTC AGT CCA GTT GAC GAC CAC TAC TCT TGG GAG GGC GAG ACT CTT GCC 1721
Leu Ser Pro Val Asp Asp His Tyr Ser Trp Glu Gly Glu Thr Leu Ala
    410                 415                 420

TCT CCT GCC ACC GAG ATC GAG TCT GGA AGG CAT TTC TGATCCTGCT 1767
Ser Pro Ala Thr Glu Ile Glu Ser Gly Arg His Phe
425                 430                 435

CAATGAGCCT TGATACGTAC TACACTGTGT ACGTGCTACT GCATTGACTA ATGAGACGGC 1827

GTTTTCAAAC AAATTTTAAC GACATGCTTG GTTATCGCAT TGAGCTGATT TCGACACATA 1887

TATATGTTTA ATACGTTTTG GGGACACTCC AGGGATTCAT GACGGTTGCT TCATATCCCG 1947

ACCTGGGGAT GGATTGACCT GGTTGTGCCC AATTTTCTTC TGCCTAACGT TTTGATTATA 2007

CATGCATTTT TCACGAAACC AGCCGGCCCG CCATGATCGT GACCTCAATT TGAGCTCGAA 2067

TCTTCCTGGG GCCTCCAGCG ATAATTCTTA ATGCTCGTTC CGAGGGTGCC ACATCGGACA 2127

TTCGCTTGTA CAACTTTTGC AGAACGAACA TTTTCACCGA TTCCAACTTG AGTCATTCGC 2187

TTACTACTTT CAACTGGTCG AGAAACTTCG CTTCTTTTCA GCTCGGCTAG GTGCATAAAT 2247

ATTTTACATT CGTGTCGATC GCTCACATTT CACGGCGCCT GGAAACTTGG GGGTTTCGAT 2307

TTCATTGGAA AGGATAAACA ACATGGGCTG GGCGCCTTTT ACACGCACTA CATTCGCTTA 2367

GAAAAGTTCT GATGCTTTTA ATGATTCTTG CATTGGCATA TAGAAAGGGG TCCTCCAGAC 2427

TCGCTACTGT GGTCCTCTCT CAACCCCACA CTCGCTTGCT TTTAACAGTG GACACCCCGT 2487

GGAGCTACGT CTCCATCAAA TATTTGGCAT CAACCGGAAT CGATGCCAGG AGGACTGAGC 2547

TTACTCACGG TGACCGTCGG GTAAAAGGCG TTCTAACAGA ACATCTCCTC TATCCTCCTG 2607

TCCCATCTCG ATTCTCTGGC TGCTGGACGC AACACACCTC GCTGCACGTT TTCGACTTCC 2667

TAATACGACC TAATATCATT CTCGGTTTTC TTTGCTCTGG CTCGCGGCCG CCATTTATAT 2727

GGCGTGTCGG CTCGAGTCTG AAGTGAACTT TTTTCTCCTT TCTGGCCTCC ACAACTTTCC 2787

GATCCCTAGC AGCTTCCCGT GCACAGCGAG GTGTTGTTGG ATGATTGTTC CATAGCATTA 2847

TCATTATTCC TAATCCGGTA GCGTTATGAT TTATGAAGAA CAGTGATGTA CATTATTATG 2907

CGGTGACTAA AAAAAAAAAA AAAAAAAA 2935

(2) INFORMATION FOR SEQ ID NO:14:

(i) SEQUENCE CHARACTERISTICS:
(A) LENGTH: 436 amino acids
(B) TYPE: amino acid
(D) TOPOLOGY: linear

(ii) MOLECULE TYPE: protein

(xi) SEQUENCE DESCRIPTION: SEQ ID NO:14:

Met Asn Thr Thr Leu Pro Ser Trp Lys Asp Arg Thr Gln Asn Gln Phe
1               5                   10                  15

Gly Lys Leu Gln Ile Gln Val Pro Trp Arg Thr Ile Gln Leu Leu Val
            20                  25                  30

Pro His Arg Met Arg Arg Lys Ile Arg Ser Lys Leu Arg Ser Arg Ile
        35                  40                  45

Ser Pro Thr Ser Ser Ile Ser Ser Leu Gln Thr Ser Phe Ser Pro Val
    50                  55                  60

Asp Thr Leu Arg Ser Leu Gln Ser His Arg Trp Thr Leu Tyr Asp Phe
65                  70                  75                  80

Gln Tyr Leu Leu Leu Leu Ile Val Gly Ile Phe Ser Leu Ser Val Met
                85                  90                  95

Glu Ser Pro Gly Pro Leu Ala Lys Thr Ala Ala Phe Thr Leu Leu Leu
            100                 105                 110

Val Ser Leu Leu Leu Pro Ile Thr Arg Gln Phe Phe Leu Pro Phe Leu
        115                 120                 125

Pro Ile Ala Gly Trp Leu Ile Phe Phe Tyr Ala Cys Gln Phe Ile Pro
    130                 135                 140

Ser Asp Trp Arg Pro Ala Ile Trp Val Arg Val Leu Pro Ala Leu Glu
145                 150                 155                 160

Asn Ile Leu Tyr Gly Ala Asn Ile Ser Asn Ile Leu Ser Ala His Gln
                165                 170                 175

Asn Val Val Leu Asp Val Leu Ala Trp Leu Pro Tyr Gly Ile Cys His
            180                 185                 190

Tyr Gly Ala Pro Phe Val Cys Ser Ala Ile Met Phe Ile Phe Gly Pro
        195                 200                 205

Pro Gly Thr Val Pro Leu Phe Ala Arg Thr Phe Gly Tyr Ile Ser Met
    210                 215                 220

Ala Ala Val Thr Ile Gln Leu Phe Phe Pro Cys Ser Pro Pro Trp Tyr
225                 230                 235                 240

Glu Asn Leu Tyr Gly Leu Ala Pro Ala Asp Tyr Ser Met Pro Gly Asn
                245                 250                 255

Pro Ala Gly Leu Ala Arg Ile Asp Glu Leu Phe Gly Ile Asp Leu Tyr
            260                 265                 270

Thr Ser Gly Phe Arg Gln Ser Pro Val Val Phe Gly Ala Phe Pro Ser
        275                 280                 285

Leu His Ala Ala Asp Ser Thr Leu Ala Ala Leu Phe Met Ser Gln Val
    290                 295                 300

Phe Pro Arg Leu Lys Pro Leu Phe Val Ile Tyr Thr Leu Trp Met Trp
305                 310                 315                 320

Trp Ala Thr Met Tyr Leu Ser His His Tyr Ala Val Asp Leu Val Gly
                325                 330                 335

Gly Gly Leu Leu Ala Thr Val Ala Phe Tyr Phe Ala Lys Thr Arg Phe
            340                 345                 350

Met Pro Arg Val Gln Asn Asp Lys Met Phe Arg Trp Asp Tyr Asp Tyr
        355                 360                 365

Val Glu Ser Gly Asp Ser Ala Leu Asp Tyr Gly Tyr Gly Pro Ala Ser
    370                 375                 380

Phe Glu Gly Glu Phe Asn Leu Asp Ser Asp Glu Trp Thr Val Gly Ser
385                 390                 395                 400

Ser Ser Ser Ile Ser Ser Gly Ser Leu Ser Pro Val Asp Asp His Tyr
                405                 410                 415

Ser Trp Glu Gly Glu Thr Leu Ala Ser Pro Ala Thr Glu Ile Glu Ser
            420                 425                 430

Gly Arg His Phe
        435

(2) INFORMATION FOR SEQ ID NO:15:

(i) SEQUENCE CHARACTERISTICS:
(A) LENGTH: 2959 base pairs
(B) TYPE: nucleic acid
(C) STRANDEDNESS: single
(D) TOPOLOGY: linear

(ii) MOLECULE TYPE: RNA (genomic)

(iii) HYPOTHETICAL: NO

(iv) ANTI-SENSE: NO

(xi) SEQUENCE DESCRIPTION: SEQ ID NO:15:

AUAUGAUUUA AAAUUAAUGU UUCCAUUUUC UUCCCCAUAA CAACUCUUCU CGCCCUUCCU 60

CCGGCUCCGU GGCCAAAUUG UUUUAUGCAG CGCCUCCUAG CGAUUUAACC UCGUUCUCGU 120

UGCCCUUGCC UGUCCGCCUU GCGUCAGUAC GACCCUUGCA ACGUGACCUU CCCCAGAGUA 180

UCCUCGUUUG GCCGCUGGAG ACCGGAGCUU GCACCCUCAU AAACUAGCUC UUCGAAAUCA 240

AUUCUCCGUU CUCCAGAGAU UAUCGGAUCG AAUCUCUCCG CUGUCGACAC CUUUCGUCUC 300

UCGGUGAUCC UCGCCCUUGG AGUCUCGUCA CGUUGACGCC UUGAACCCCU GGCCGCCAAC 360

UCCACAUAGG AGACCACACU UCAUUCUUCC CCCGCCAUAA UUGCAGCACC CUCCGUCUCC 420

CUUCGAGCUC CUCCUGGAUC AUCAAGUCCG AAAGGAUUAG ACUCGUCGCA GCGAUGAAUA 480

CCACCCUUCC AUCCUGGAAG GAUCGGACGC AAAACCAGUU CGGCAAGCUC CAGAUCCAAG 540

UCCCAUGGCG CACCAUACAA CUUCUCGUGC CGCACCGUAU GCGACGGAAG AUUCGGUCCA 600

AGCUGCGCAG UCGGAUCUCG CCUACCUCAU CGAUAUCCUC GUUGCAGACG UCAUUCUCAC 660

CUGUCGAUAC ACUCAGGUCG CUGCAAAGUC AUAGAUGGAC GCUCUAUGAC UUUCAGUAUC 720

UUUUGCUGCU GAUUGUCGGC AUAUUCUCGC UGAGCGUUAU GGAAUCACCU GGACCAUUGG 780

CAAAGACCGC CGCGUUUACG CUACUUCUCG UCUCUCUCCU UCUCCCGAUU ACGCGCCAGU 840

UCUUCUUGCC AUUCCUCCCG AUUGCAGGAU GGCUUAUAUU UUUCUACGCU UGCCAGUUCA 900

UCCCGAGCGA CUGGCGCCCU GCAAUCUGGG UUCGCGUGCU GCCGGCUCUG GAAAACAUUC 960

UCUACGGUGC UAAUAUCAGU AACAUCCUUU CCGCUCACCA AAAUGUGGUG CUUGACGUUU 1020

UGGCGUGGCU UCCCUACGGA AUCUGCCAUU AUGGCGCGCC AUUUGUGUGC UCAGCGAUCA 1080

UGUUCAUCUU UGGUCCUCCC GGCACCGUCC CCCUUUUCGC UCGAACUUUU GGAUACAUCA 1140

GCAUGGCUGC AGUCACCAUU CAGCUGUUUU UCCCCUGCUC UCCUCCGUGG UACGAAAAUC 1200

UGUAUGGUUU GGCUCCGGCU GAUUACUCCA UGCCGGGUAA UCCUGCGGGC CUUGCUCGCA 1260

UCGAUGAGCU UUUUGGGAUA GACUUGUACA CAUCGGGCUU CAGACAAUCU CCCGUCGUGU 1320

UUGGCGCAUU UCCUUCCCUA CAUGCCGCUG AUUCGACACU UGCAGCUCUA UUUAUGAGCC 1380

AAGUGUUCCC ACGGUUGAAG CCCUUGUUUG UCAUCUAUAC UCUCUGGAUG UGGUGGGCUA 1440

CAAUGUAUCU UUCGCACCAC UACGCUGUUG AUCUGGUCGG UGGUGGCCUC UUGGCAACUG 1500

UCGCGUUCUA CUUUGCUAAA ACGCGGUUCA UGCCUCGCGU CCAGAAUGAU AAGAUGUUCC 1560

GCUGGGACUA CGAUUAUGUU GAGUCAGGCG AUUCCGCACU CGACUAUGGG UACGGUCCAG 1620

CCAGCUUCGA AGGCGAAUUC AACCUUGAUA GCGAUGAGUG GACCGUUGGU UCUUCGUCAU 1680

CCAUUUCGUC CGGCUCCCUC AGUCCAGUUG ACGACCACUA CUCUUGGGAG GGCGAGACUC 1740

UUGCCUCUCC UGCCACCGAG AUCGAGUCUG GAAGGCAUUU CUGAUCCUGC UCAAUGAGCC 1800

UUGAUACGUA CUACACUGUG UACGUGCUAC UGCAUUGACU AAUGAGACGG CGUUUUCAAA 1860

CAAAUUUUAA CGACAUGCUU GGUUAUCGCA UUGAGCUGAU UUCGACACAU AUAUAUGUUU 1920

AAUACGUUUU GGGGACACUC CAGGGAUUCA UGACGGUUGC UUCAUAUCCC GACCUGGGGA 1980

UGGAUUGACC UGGUUGUGCC CAAUUUUCUU CUGCCUAACG UUUUGAUUAU ACAUGCAUUU 2040

UUCACGAAAC CAGCCGGCCC GCCAUGAUCG UGACCUCAAU UUGAGCUCGA AUCUUCCUGG 2100

GGCCUCCAGC GAUAAUUCUU AAUGCUCGUU CCGAGGGUGC CACAUCGGAC AUUCGCUUGU 2160

ACAACUUUUG CAGAACGAAC AUUUUCACCG AUUCCAACUU GAGUCAUUCG CUUACUACUU 2220

UCAACUGGUC GAGAAACUUC GCUUCUUUUC AGCUCGGCUA GGUGCAUAAA UAUUUUACAU 2280

UCGUGUCGAU CGCUCACAUU UCACGGCGCC UGGAAACUUG GGGGUUUCGA UUUCAUUGGA 2340

AAGGAUAAAC AACAUGGGCU GGGCGCCUUU UACACGCACU ACAUUCGCUU AGAAAAGUUC 2400

UGAUGCUUUU AAUGAUUCUU GCAUUGGCAU AUAGAAAGGG GUCCUCCAGA CUCGCUACUG 2460

UGGUCCUCUC UCAACCCCAC ACUCGCUUGC UUUUAACAGU GGACACCCCG UGGAGCUACG 2520

UCUCCAUCAA AUAUUUGGCA UCAACCGGAA UCGAUGCCAG GAGGACUGAG CUUACUCACG 2580

GUGACCGUCG GGUAAAAGGC GUUCUAACAG AACAUCUCCU CUAUCCUCCU GUCCCAUCUC 2640

GAUUCUCUGG CUGCUGGACG CAACACACCU CGCUGCACGU UUUCGACUUC CUAAUACGAC 2700

CUAAUAUCAU UCUCGGUUUU CUUUGCUCUG GCUCGCGGCC GCCAUUUAUA UGGCGUGUCG 2760

GCUCGAGUCU GAAGUGAACU UUUUUCUCCU UUCUGGCCUC CACAACUUUC CGAUCCCUAG 2820

CAGCUUCCCG UGCACAGCGA GGUGUUGUUG GAUGAUUGUU CCAUAGCAUU AUCAUUAUUC 2880

CUAAUCCGGU AGCGUUAUGA UUUAUGAAGA ACAGUGAUGU ACAUUAUUAU GCGGUGACUA 2940

AAAAAAAAAA AAAAAAAAA 2959

(2) INFORMATION FOR SEQ ID NO:16:

(i) SEQUENCE CHARACTERISTICS:
(A) LENGTH: 2856 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(ix) FEATURE:
(A) NAME/KEY: CDS
(B) LOCATION: 395..1715

(xi) SEQUENCE DESCRIPTION: SEQ ID NO:16:

GGTTTATACT CCGGCTCCGT GGCCATCTGC CTCCCTCACG ACCTCCTCGT TCCAGGTTTT 60

CCTCTCGACT GCTGCGCCCT TGCACTTCGC CTTGCATCAG TGAAACCCCC TGCAACGTGA 120

CGGCTCAAAG ACATCCTCGT TTGGCCGCTG GAGACCGGAG CGTGCGCTTC GTTTCGTCTT 180

CTTCGAACCG ATCTCAATTT CCCCGCTCGG GTTGACGCCG TCAGCACCCT GCTCGTTGCC 240

TCCCGGCTTG TTATTCAAGA CCCCTTTTCT GCCGCTTCCG CGACCGATTT ATTCGTCGCC 300

TTCCAACTCT TGTACAATCG GGGGGAAAGA AAGCAGACGG AGTTCGATCT GGAGGAATTA 360

TAGCTGAGTC TTGCCCGCAA GACTCGCCGC AACC ATG AAT CAA ACA CTT CCC 412
Met Asn Gln Thr Leu Pro
1               5

ACG TGG AAG GAC CGC ACG GAC AAC CAG TTT GGA AAG CTT CAG ATC CAG 460
Thr Trp Lys Asp Arg Thr Asp Asn Gln Phe Gly Lys Leu Gln Ile Gln
            10                  15                  20

GTT CCA TGG CGG TCC ATC CAA CTG CTC GTC CCG CAT CGC ATG CGG CGG 508
Val Pro Trp Arg Ser Ile Gln Leu Leu Val Pro His Arg Met Arg Arg
        25                  30                  35

AAG TTA AGG TCC AAA TTG CGC AGT AGA GCG TCT CCT ACC TCG TCA ATA 556
Lys Leu Arg Ser Lys Leu Arg Ser Arg Ala Ser Pro Thr Ser Ser Ile
    40                  45                  50

GCC TCT TTA CAG ACG TCG TTA TCG CCT GCA GAC ACA CTA CGA TCG CTC 604
Ala Ser Leu Gln Thr Ser Leu Ser Pro Ala Asp Thr Leu Arg Ser Leu
55                  60                  65                  70

CAA AGC CAC CGA TGG ACG GTT TAC GAC TTC CAA TAT CTG CTT CTG TTG 652
Gln Ser His Arg Trp Thr Val Tyr Asp Phe Gln Tyr Leu Leu Leu Leu
                75                  80                  85

ATC GTG GGC ATC TTC TCT TTG ACC GTT ATC GAG TCG CCC GGG CCT TTG 700
Ile Val Gly Ile Phe Ser Leu Thr Val Ile Glu Ser Pro Gly Pro Leu
            90                  95                  100

GGC AAA ACG GCC ATT TTC TCC ATG CTC CTA TTC TCT CTC CTG ATC CCT 748
Gly Lys Thr Ala Ile Phe Ser Met Leu Leu Phe Ser Leu Leu Ile Pro
        105                 110                 115

ATG ACC CGC CAG TTC TTC CTC CCG TTT CTG CCG ATT GCC GGA TGG CTT 796
Met Thr Arg Gln Phe Phe Leu Pro Phe Leu Pro Ile Ala Gly Trp Leu
    120                 125                 130

CTG TTT TTC TAC GCC TGC CAG TTC ATC CCA AGC GAT TGG CGC CCT GCG 844
Leu Phe Phe Tyr Ala Cys Gln Phe Ile Pro Ser Asp Trp Arg Pro Ala
135                 140                 145                 150

ATT TGG GTT CGT GTC TTG CCT GCA CTG GAG AAT ATT CTC TAC GGC GCA 892
Ile Trp Val Arg Val Leu Pro Ala Leu Glu Asn Ile Leu Tyr Gly Ala
                155                 160                 165

AAC ATC AGC AAC ATC CTA TCC GCT CAC CAG AAC GTT GTG CTT GAC GTG 940
Asn Ile Ser Asn Ile Leu Ser Ala His Gln Asn Val Val Leu Asp Val
            170                 175                 180

CTG GCG TGG CTA CCC TAC GGT ATC TGC CAC TAT GGC GCT CCG TTT GTG 988
Leu Ala Trp Leu Pro Tyr Gly Ile Cys His Tyr Gly Ala Pro Phe Val
        185                 190                 195

TGC TCG TTG ATC ATG TTC ATC TTC GGT CCG CCC GGC ACT GTT CCC CTT 1036
Cys Ser Leu Ile Met Phe Ile Phe Gly Pro Pro Gly Thr Val Pro Leu
    200                 205                 210

TTC GCG CGC ACT TTC GGC TAT ATC AGT ATG ACT GCG GTT ACT ATT CAG 1084
Phe Ala Arg Thr Phe Gly Tyr Ile Ser Met Thr Ala Val Thr Ile Gln
215                 220                 225                 230

CTG TTT TTC CCT TGC TCT CCA CCT TGG TAT GAG AAT CGC TAT GGT CTA 1132
Leu Phe Phe Pro Cys Ser Pro Pro Trp Tyr Glu Asn Arg Tyr Gly Leu
                235                 240                 245

GCT CCG GCA GAC TAC TCC ATC CAA GGT GAT CCC GCA GGG CTT GCC CGC 1180
Ala Pro Ala Asp Tyr Ser Ile Gln Gly Asp Pro Ala Gly Leu Ala Arg
            250                 255                 260

ATT GAC AAG CTT TTC GGC ATC GAC CTT TAC ACG TCT GTT TTC CAT CAG 1228
Ile Asp Lys Leu Phe Gly Ile Asp Leu Tyr Thr Ser Val Phe His Gln
        265                 270                 275

TCG CCT GTT GTG TTC GGC GCT TTT CCG TCG CTG CAT GCT GCC GAC TCA 1276
Ser Pro Val Val Phe Gly Ala Phe Pro Ser Leu His Ala Ala Asp Ser
    280                 285                 290

ACC CTG GCC GCA CTT TTC ATG AGT CAT GTT TTC CCC CGC ATG AAG CCC 1324
Thr Leu Ala Ala Leu Phe Met Ser His Val Phe Pro Arg Met Lys Pro
295                 300                 305                 310

GTC TTC GTG ACC TAT ACT CTA TGG ATG TGG TGG GCA ACA ATG TAC CTC 1372
Val Phe Val Thr Tyr Thr Leu Trp Met Trp Trp Ala Thr Met Tyr Leu
                315                 320                 325

TCA CAT CAC TAT GCG GTC GAT TTG GTT GCG GGT GGT CTC CTG GCC GCC 1420
Ser His His Tyr Ala Val Asp Leu Val Ala Gly Gly Leu Leu Ala Ala
            330                 335                 340

ATT GCT TTC TAC TTC GCC AAG ACC CGA TTC CTT CCC CGT GTC CAG CTC 1468
Ile Ala Phe Tyr Phe Ala Lys Thr Arg Phe Leu Pro Arg Val Gln Leu
        345                 350                 355

GAC AAG ACC TTC CGT TGG GAC TAC GAC TAT GTG GAA TTC GGC GAG TCT 1516
Asp Lys Thr Phe Arg Trp Asp Tyr Asp Tyr Val Glu Phe Gly Glu Ser
    360                 365                 370

GCC CTG GAG TAT GGG TAT GGT GCA GCT GGC TAT GAT GGA GAC TTC AAT 1564
Ala Leu Glu Tyr Gly Tyr Gly Ala Ala Gly Tyr Asp Gly Asp Phe Asn
375                 380                 385                 390

CTC GAC AGC GAT GAA TGG ACT GTT GGT TCT TCA TCC TCC GTC TCC TCA 1612
Leu Asp Ser Asp Glu Trp Thr Val Gly Ser Ser Ser Ser Val Ser Ser
                395                 400                 405

GGC TCC TTG AGT CCC GTT GAC GAT CAT TAC TCA TGG GAA ACC GAG GCA 1660
Gly Ser Leu Ser Pro Val Asp Asp His Tyr Ser Trp Glu Thr Glu Ala
            410                 415                 420

CTG ACC TCC CCA CAT ACT GAT ATT GAG TCC GGC AGG CAT ACT TTC AGC 1708

Leu Thr Ser Pro His Thr Asp Ile Glu Ser Gly Arg His Thr Phe Ser
        425                 430                 435

CCT TGA G TAGCCACAAA CCAAACTCGA TACCTGCATA TAGCGATCTC GCTCCTCCTC     1765
Pro  *
    440

CACTGCATCT ATTTACGAGA CGGCGTTAGA ACATTTCACG ACATTCTGGC TTTATTGCAT     1825

CGAGCACATT TCGACACATA TATCTTTAAT ACCCTTTCTT CGGTGTCCCA GATCATCGGT     1885

TCGACCTTAA TGTACCTCGG TCCGAATCCG CCTGGGATAC TGTTTCTCTT TCCGCCGCAC     1945

TTCACTGTAC ATTGCTTGAC ATTGCGAAAC CGGGTTGGGC TCGAACGTGG GATGGGTTAT     2005

CGCTCATCGC TACACGCCGT TGCTCCATCA TAATGTTAAT GGACACAATG GGGCTACGCA     2065

TCCTGGTGTT TAGTCCTGGA AGACCATCCG ATAACCCCCG TCGGTAACAC TCGCTTGTCT     2125

CGTGTCCACC CAGACACTAC TTCAATTCTC ACTTCTATCG TCCGCTATTA CCTTGACCTG     2185

GTCGAACCCA TCCTTATTAT TCGTTTCGAC TATGCTATAT ATTTATTTTT ACCATTCGTG     2245

TCGATCGCTC ATACTCTTGG CGCTTGGGAC TGGAAGCATT TATATTGGAA AAAATCACGG     2305

AATGGGGCGC CTTTTCTTCT TGCACTTCAC TCGCTGTGCA TAGACGGTTT TACATTTCTG     2365

CTTTGCAATG CATCACGAAC TCTGCATTAG CATATAGAAA GAGGGGAAGG ATGGACCTTC     2425

TTCTTGATTG CTCGCATGGT TTATCCATTC GCTCAAAGTG GATTACGTCC ACATCTTACC     2485

CGGGGGCTAT ACACATGGCT ACTGTGTTGC TTTCTGACAT TCGCCGGACG TGCAAGGTTG     2545

GGAGGAGAGT CTGACGCTGA CGGGGCTTGT TGAAGGATGT TCACGCGTCC CGATTTGACC     2605

CGGCTTCGAC TAACCTCAGA TTCTCGACTT GTTGGACGGT GACTTGACTT GCTTGCTATG     2665

GTCTGACGCT CTCACACCTA CCTATCACAT CCTCCTCACC TCACAAATTC CGCTCATGGA     2725

CACTATCCTC TTCTTTTCGT TTCCCTTGGA TAGTGTGTGT GTGTGTGTGG TTGGGGCAAA     2785

TTATCCATAG CAGCAGTATT ATTAGTTATA ATCCGGTAGT GTTATGATTT ATGAAGGCAA     2845

CTTGTATACT A                                                          2856

(2) INFORMATION FOR SEQ ID NO:17:

     (i) SEQUENCE CHARACTERISTICS:
          (A) LENGTH: 440 amino acids
          (B) TYPE: amino acid
          (D) TOPOLOGY: linear

    (ii) MOLECULE TYPE: protein

    (xi) SEQUENCE DESCRIPTION: SEQ ID NO:17:

Met Asn Gln Thr Leu Pro Thr Trp Lys Asp Arg Thr Asp Asn Gln Phe
  1               5                  10                  15

Gly Lys Leu Gln Ile Gln Val Pro Trp Arg Ser Ile Gln Leu Leu Val
             20                  25                  30

Pro His Arg Met Arg Arg Lys Leu Arg Ser Lys Leu Arg Ser Arg Ala
         35                  40                  45

Ser Pro Thr Ser Ser Ile Ala Ser Leu Gln Thr Ser Leu Ser Pro Ala
        50                  55                  60

Asp Thr Leu Arg Ser Leu Gln Ser His Arg Trp Thr Val Tyr Asp Phe
    65                  70                  75                  80

Gln Tyr Leu Leu Leu Leu Ile Val Gly Ile Phe Ser Leu Thr Val Ile
                85                  90                  95

Glu Ser Pro Gly Pro Leu Gly Lys Thr Ala Ile Phe Ser Met Leu Leu
            100                 105                 110

Phe Ser Leu Leu Ile Pro Met Thr Arg Gln Phe Phe Leu Pro Phe Leu
        115                 120                 125

Pro Ile Ala Gly Trp Leu Leu Phe Phe Tyr Ala Cys Gln Phe Ile Pro
    130                 135                 140

Ser Asp Trp Arg Pro Ala Ile Trp Val Arg Val Leu Pro Ala Leu Glu
145                 150                 155                 160

Asn Ile Leu Tyr Gly Ala Asn Ile Ser Asn Ile Leu Ser Ala His Gln
                165                 170                 175

Asn Val Val Leu Asp Val Leu Ala Trp Leu Pro Tyr Gly Ile Cys His
            180                 185                 190

Tyr Gly Ala Pro Phe Val Cys Ser Leu Ile Met Phe Ile Phe Gly Pro
        195                 200                 205

Pro Gly Thr Val Pro Leu Phe Ala Arg Thr Phe Gly Tyr Ile Ser Met
    210                 215                 220

Thr Ala Val Thr Ile Gln Leu Phe Phe Pro Cys Ser Pro Pro Trp Tyr
225                 230                 235                 240

Glu Asn Arg Tyr Gly Leu Ala Pro Ala Asp Tyr Ser Ile Gln Gly Asp
                245                 250                 255

Pro Ala Gly Leu Ala Arg Ile Asp Lys Leu Phe Gly Ile Asp Leu Tyr
            260                 265                 270

Thr Ser Val Phe His Gln Ser Pro Val Val Phe Gly Ala Phe Pro Ser
        275                 280                 285

Leu His Ala Ala Asp Ser Thr Leu Ala Ala Leu Phe Met Ser His Val
    290                 295                 300

Phe Pro Arg Met Lys Pro Val Phe Val Thr Tyr Thr Leu Trp Met Trp
305                 310                 315                 320

Trp Ala Thr Met Tyr Leu Ser His His Tyr Ala Val Asp Leu Val Ala
                325                 330                 335

Gly Gly Leu Leu Ala Ala Ile Ala Phe Tyr Phe Ala Lys Thr Arg Phe
            340                 345                 350

Leu Pro Arg Val Gln Leu Asp Lys Thr Phe Arg Trp Asp Tyr Asp Tyr
        355                 360                 365

Val Glu Phe Gly Glu Ser Ala Leu Glu Tyr Gly Tyr Gly Ala Ala Gly
    370                 375                 380

Tyr Asp Gly Asp Phe Asn Leu Asp Ser Asp Glu Trp Thr Val Gly Ser

385                 390                 395                 400

Ser Ser Ser Val Ser Ser Gly Ser Leu Ser Pro Val Asp Asp His Tyr
                405                 410                 415

Ser Trp Glu Thr Glu Ala Leu Thr Ser Pro His Thr Asp Ile Glu Ser
            420                 425                 430

Gly Arg His Thr Phe Ser Pro  *
        435                 440

(2) INFORMATION FOR SEQ ID NO:18:

     (i) SEQUENCE CHARACTERISTICS:
          (A) LENGTH: 2877 base pairs
          (B) TYPE: nucleic acid
          (C) STRANDEDNESS: single
          (D) TOPOLOGY: linear

    (ii) MOLECULE TYPE: RNA (genomic)

   (iii) HYPOTHETICAL: NO

    (iv) ANTI-SENSE: NO

    (xi) SEQUENCE DESCRIPTION: SEQ ID NO:18:

UAAAAUUUCA UAAAUGUUUC CGGUUUAUAC UCCGGCUCCG UGGCCAUCUG CCUCCCUCAC      60

GACCUCCUCG UUCCAGGUUU UCCUCUCGAC UGCUGCGCCC UUGCACUUCG CCUUGCAUCA     120

GUGAAACCCC CUGCAACGUG ACGGCUCAAA GACAUCCUCG UUUGGCCGCU GGAGACCGGA     180

GCGUGCGCUU CGUUUCGUCU UCUUCGAACC GAUCUCAAUU UCCCCGCUCG GGUUGACGCC     240

GUCAGCACCC UGCUCGUUGC CUCCCGGCUU GUUAUUCAAG ACCCCUUUUC UGCCGCUUCC     300

GCGACCGAUU UAUUCGUCGC CUUCCAACUC UUGUACAAUC GGGGGGAAAG AAAGCAGACG     360

GAGUUCGAUC UGGAGGAAUU AUAGCUGAGU CUUGCCCGCA AGACUCGCCG CAACCAUGAA     420

UCAAACACUU CCCACGUGGA AGGACCGCAC GGACAACCAG UUUGGAAAGC UUCAGAUCCA     480

GGUUCCAUGG CGGUCCAUCC AACUGCUCGU CCCGCAUCGC AUGCGGCGGA AGUUAAGGUC     540

CAAAUUGCGC AGUAGAGCGU CUCCUACCUC GUCAAUAGCC UCUUUACAGA CGUCGUUAUC     600

GCCUGCAGAC ACACUACGAU CGCUCCAAAG CCACCGAUGG ACGGUUUACG ACUUCCAAUA     660

UCUGCUUCUG UUGAUCGUGG GCAUCUUCUC UUUGACCGUU AUCGAGUCGC CCGGGCCUUU     720

GGGCAAAACG GCCAUUUUCU CCAUGCUCCU AUUCUCUCUC CUGAUCCCUA UGACCCGCCA     780

GUUCUUCCUC CCGUUUCUGC CGAUUGCCGG AUGGCUUCUG UUUUUCUACG CCUGCCAGUU     840

CAUCCCAAGC GAUUGGCGCC CUGCGAUUUG GGUUCGUGUC UUGCCUGCAC UGGAGAAUAU     900

UCUCUACGGC GCAAACAUCA GCAACAUCCU AUCCGCUCAC CAGAACGUUG UGCUUGACGU     960

GCUGGCGUGG CUACCCUACG GUAUCUGCCA CUAUGGCGCU CCGUUUGUGU GCUCGUUGAU    1020

CAUGUUCAUC UUCGGUCCGC CCGGCACUGU UCCCCUUUUC GCGCGCACUU UCGGCUAUAU    1080

CAGUAUGACU GCGGUUACUA UUCAGCUGUU UUUCCCUUGC UCUCCACCUU GGUAUGAGAA 1140

UCGCUAUGGU CUAGCUCCGG CAGACUACUC CAUCCAAGGU GAUCCCGCAG GGCUUGCCCG 1200

CAUUGACAAG CUUUUCGGCA UCGACCUUUA CACGUCUGUU UUCCAUCAGU CGCCUGUUGU 1260

GUUCGGCGCU UUUCCGUCGC UGCAUGCUGC CGACUCAACC CUGGCCGCAC UUUUCAUGAG 1320

UCAUGUUUUC CCCCGCAUGA AGCCCGUCUU CGUGACCUAU ACUCUAUGGA UGUGGUGGGC 1380

AACAAUGUAC CUCUCACAUC ACUAUGCGGU CGAUUUGGUU GCGGGUGGUC UCCUGGCCGC 1440

CAUUGCUUUC UACUUCGCCA AGACCCGAUU CCUUCCCCGU GUCCAGCUCG ACAAGACCUU 1500

CCGUUGGGAC UACGACUAUG UGGAAUUCGG CGAGUCUGCC CUGGAGUAUG GGUAUGGUGC 1560

AGCUGGCUAU GAUGGAGACU UCAAUCUCGA CAGCGAUGAA UGGACUGUUG GUUCUUCAUC 1620

CUCCGUCUCC UCAGGCUCCU UGAGUCCCGU UGACGAUCAU UACUCAUGGG AAACCGAGGC 1680

ACUGACCUCC CCACAUACUG AUAUUGAGUC CGGCAGGCAU ACUUUCAGCC CUUGAGUAGC 1740

CACAAACCAA ACUCGAUACC UGCAUAUAGC GAUCUCGCUC CUCCUCCACU GCAUCUAUUU 1800

ACGAGACGGC GUUAGAACAU UUCACGACAU UCUGGCUUUA UUGCAUCGAG CACAUUUCGA 1860

CACAUAUAUC UUUAAUACCC UUUCUUCGGU GUCCCAGAUC AUCGGUUCGA CCUUAAUGUA 1920

CCUCGGUCCG AAUCCGCCUG GGAUACUGUU UCUCUUUCCG CCGCACUUCA CUGUACAUUG 1980

CUUGACAUUG CGAAACCGGG UUGGGCUCGA ACGUGGGAUG GGUUAUCGCU CAUCGCUACA 2040

CGCCGUUGCU CCAUCAUAAU GUUAAUGGAC ACAAUGGGGC UACGCAUCCU GGUGUUUAGU 2100

CCUGGAAGAC CAUCCGAUAA CCCCCGUCGG UAACACUCGC UUGUCUCGUG UCCACCCAGA 2160

CACUACUUCA AUUCUCACUU CUAUCGUCCG CUAUUACCUU GACCUGGUCG AACCCAUCCU 2220

UAUUAUUCGU UUCGACUAUG CUAUAUAUUU AUUUUUACCA UUCGUGUCGA UCGCUCAUAC 2280

UCUUGGCGCU UGGGACUGGA AGCAUUUAUA UUGGAAAAAA UCACGGAAUG GGGCGCCUUU 2340

UCUUCUUGCA CUUCACUCGC UGUGCAUAGA CGGUUUUACA UUUCUGCUUU GCAAUGCAUC 2400

ACGAACUCUG CAUUAGCAUA UAGAAAGAGG GGAAGGAUGG ACCUUCUUCU UGAUUGCUCG 2460

CAUGGUUUAU CCAUUCGCUC AAAGUGGAUU ACGUCCACAU CUUACCCGGG GGCUAUACAC 2520

AUGGCUACUG UGUUGCUUUC UGACAUUCGC CGGACGUGCA AGGUUGGGAG GAGAGUCUGA 2580

CGCUGACGGG GCUUGUUGAA GGAUGUUCAC GCGUCCCGAU UUGACCCGGC UUCGACUAAC 2640

CUCAGAUUCU CGACUUGUUG GACGGUGACU UGACUUGCUU GCUAUGGUCU GACGCUCUCA 2700

CACCUACCUA UCACAUCCUC CUCACCUCAC AAAUUCCGCU CAUGGACACU AUCCUCUUCU 2760

UUUCGUUUCC CUUGGAUAGU GUGUGUGUGU GUGUGGUUGG GGCAAAUUAU CCAUAGCAGC 2820

AGUAUUAUUA GUUAUAAUCC GGUAGUGUUA UGAUUUAUGA AGGCAACUUG UAUACUA 2877

(2) INFORMATION FOR SEQ ID NO:19:

(i) SEQUENCE CHARACTERISTICS:
(A) LENGTH: 3220 base pairs

(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(ix) FEATURE:
(A) NAME/KEY: intron
(B) LOCATION: 1888..1939

(xi) SEQUENCE DESCRIPTION: SEQ ID NO:19:

GTCGACATAG TTACACTCCC CTCTACTCTT CAGACTACCC CTTCTGATTA TCAGGAGACT 60

ACGACTACTC CGCCTACCTT GACCTTTGCA AACTCGGAGC CACGACCGAG TCCTTCCCTC 120

CATCGTTCCC TGCGCAACAT CGGTTTGGCC GACGGCAGCA TGACTTGTGC CCCAGGCTAT 180

ATCCTCATCT GCATTCATCA TGACCCCTTG TCTCGTTACA CGCTTGGCAT CAATTTTACC 240

ACCTTTGTGG GTCTTTGCCC CACGACCCCT GCTAGTACCA GCACTTCCGG CGGTCCCGCC 300

TTTTCCTCTT TTACACCCAA GTTCGCCGAC CATATGCGAT GTCATGTCAT CCAGCGCTTG 360

CTGCACACTG TCATGCACGA CCGTCTCGCC AACATGCCGG GTTGCCACAC CTGGGAATCG 420

TTGGTCAATG CGGCGTACCG TTACTGCACC TGGGCTATGG AATGTATCTT CCTCGGCACC 480

GATGCAAATG TTCAATTCGG GATTGCTTGT TATCCCCACT TGAAAGAGCT CGAAGAGGCG 540

GCAAGGACGC TCAGGAAGGA TAGTATGGGC GAGTGGGATG ATGTGGTGCA GATTGGGGTG 600

GATAGGTTGT TGGAAATACT ACCTTGTGGT GAACGGACCT TGATCAACAA GAGAAGGATC 660

GAAAAGGAAC GACTGCAAGC GCTTCTAAAA GCTCAAACTC AAGTCAAGGC CAAGCATGAG 720

GCCAGATGTC AGGTGGCAAC CCCGATCCCA GCTCCAGCTG TCATTCCTGT CTCTGCACCT 780

GCTCCGGTTA TAATCAAGCG TAAAAAATAC AGTAGCCTAC GCAAGCTGAT GGGACGTGCG 840

GGGTCGGTGA TTGGCAGGCA GAAGATGCGG GCAAGGGCGT CTGCGATTAT CGAGAAGGGA 900

AAGGCCGGCT TCTACGATAG GCAAAAGGTC CAGCGCATGG AGCCGGCTCC ATCCATGTCT 960

CGTCCTGCGG GAGTGTCTTG TGTGTGATTG TCAGCTGGCC GTGAAGATAG TCTTGGAGCT 1020

GTGTATAGGG ATACTACATG ATGTGCATGC AGTGTGTTTT CAAAGCAATT GCCACGTGGT 1080

GTTCGCGTGG GTCGGAAACG AGGTTCGCGT TGTTATGGTT CTTGTTTTGA GATTCTCGCG 1140

CTGGCTACAC GTACGCACCA TACACTGCAG ACGACAGCGG CAAGCATACA GACGGCAACG 1200

CTGACATCCC AGCCACACAC ACCATGTCCG CCATCCGCGC ACTCACGAGC CCCGTCACCG 1260

CCTGCTTCTC CACCTCTCTC TCCCCGCATG CCGCATTCCA CCGCTTCCTC CATGCGCTCT 1320

CCGCCTCCAT CCGCCGCCTC GACCTCTCCC GCGACCCGCG CAAGACCTTG GACCGCCTCA 1380

GGCAGCACAG GTTCACCCTT GCCAACACTT TGCCCCGCGC GTTCATGCTT CTGTGCGCCT 1440
CCTACAGTCT CTACATCATG ACCACCCCGC CCTTCCCACT CAAGCTCGGG ATACCCATCG 1500
CATACATCGC CGCTGTCATC TTCCCGATCA CGTCGCAGTT TGTCTGGCCG GCGACACCCA 1560
TCTTTGCATG GCTCATCACC TTTTTCTCCG CCCGTTTCAT CCCTTCCGGC CGCCGGCCCG 1620
AAATCCACGT CGCCCTTCTC CCAGCCCTTG AATCCGTCCT CTACGGTGCC AACATTTCTG 1680
ATCTCCAAAC ACGATATACC AACGCTTTCC TTGATGTGGT CGCCTGGCTG CCCTATGGGG 1740
TATTACATTT CACCCTTCCA TTCGTCGTGG CTGTTATCCT TTGGTCACTG GGACCTAGGG 1800
GTGCGGTTCA GTTCTGGGGT TTGGCCTTTG GGTGGATGAA TTTGCTCGGT GTCGTCTGCC 1860
AGCTCCTCTT CCCTGCTGCC GCGCCTTGTA AGTACCTCTC TCTTTTATTT AATATATCCA 1920
TACTTAATAT GCCGGGCAGG GTACGAAATC ATTCACGGTC TTACCCCCGC CGACTATTCC 1980
ATGGCTGGCT CTCCCGGCGG TCTCATGCGT ATCGACCGCG TCTTCCATTC TTCAGGCTAT 2040
ACCAACGCAT TCGGTTCCGC TCCTCTTGTT TTTGGCGCCT TCCCCTCCCT CCATTCAGGA 2100
TGTGCCGTCA TGGAAGCCCT CTTCCTCTCC CATTTCTTCC CATCCCTCAA GGGCCTGTAT 2160
TGGGGCTATG TCGGTGTCCT CTGGTGGGCG ACCATGTACC TCTCCCACCA CTACCTCATC 2220
GACCTCGTTG GCGGAGCTTG TCTGAGCGTC TTGGTGTTCT ACTTGTGCAT GCCTGAAGGT 2280
TTCAAGGACG TTGATCAGAT ACAGTGGGAG GCGGTGGAAG GGGATGGATA CGAGATGATT 2340
GGCGGACCCA GAACAGGCAC AGGTCCTGAG ATTGATTTGG ATGAGGAAAT TAGAAAGTTG 2400
GAAGAACAAG GCGAAGCGCT TTTTGAGCAG GTGATAGGGG ATGAAGAGTC TCGGATCGAG 2460
ACAAGGGGAG AAGGAAACAG TGGCGCGGGC GGGAATCTGA GTGGGAATGA AAGTGGTGAT 2520
AGTGCAGATA GCGGTAGGGG AAAGGGCAAG GGGAAGGGGA AGGGGAAACA AACTGCCAAA 2580
AAGCCAAAGG CGAAGGAGCA AAGATCGGTT AGCTGGGGAG AAACAAAGGT GATGGGTGAA 2640
GGAGCGCAGG TGGCTTCAGA GAATAGCTCT TAGATATCTG TTTTAAAACA GTAGTATACC 2700
CAACAATTTT TTGACGATAT ACCCTACCAC TATGATCTTA ATAATGCAGA ATAGAAAAAC 2760
ACCTTGCCCC TCTCGCAGGC GTCAACACAT GGAGTCTAGG CAGTTAAATC ACACTACATC 2820
ATCATCGCCG AAAAAAATAA ACCTAGTAAC TCCAACCCAA CCCTCCTGGT ACAGGCCTGT 2880
TCGTCCACCG ACTGATAATT CCCATCGCCC TCGCCCTCCT CACAAGTTTA CCCACCCGCC 2940
TTTGCGACTT CCAAGTCAAC CCGGTCTCCG CCCGACTTCT TATCTTGCCC ATCGGGTTGA 3000
CGAATTCATA GGCGAAATTA GGGTTCATAT CGTGCACCAA GGGGTTCGTT TTGGTGATGT 3060
AAAAAGGATC AAGCTTGACG GCGATTTTCT TGGGAGGACC AAGAAGAGGT GGACGGGGCA 3120
AAGGTCGAGC GGTGGGGTAG ATGGATTCTT GGGTGAATGC TTGAGGGGCA GTAAACTAGA 3180
AGAGTTGAGA AAACCCAAAG GAGTCAGCCT TTATGTCGAC 3220
(2) INFORMATION FOR SEQ ID NO:20:
(i) SEQUENCE CHARACTERISTICS:

(A) LENGTH: 3168 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iv) ANTI-SENSE: NO

(ix) FEATURE:
(A) NAME/KEY: CDS
(B) LOCATION: 1224..2618

(xi) SEQUENCE DESCRIPTION: SEQ ID NO:20:

GTCGACATAG TTACACTCCC CTCTACTCTT CAGACTACCC CTTCTGATTA TCAGGAGACT 60

ACGACTACTC CGCCTACCTT GACCTTTGCA AACTCGGAGC CACGACCGAG TCCTTCCCTC 120

CATCGTTCCC TGCGCAACAT CGGTTTGGCC GACGGCAGCA TGACTTGTGC CCCAGGCTAT 180

ATCCTCATCT GCATTCATCA TGACCCCTTG TCTCGTTACA CGCTTGGCAT CAATTTTACC 240

ACCTTTGTGG GTCTTTGCCC CACGACCCCT GCTAGTACCA GCACTTCCGG CGGTCCCGCC 300

TTTTCCTCTT TTACACCCAA GTTCGCCGAC CATATGCGAT GTCATGTCAT CCAGCGCTTG 360

CTGCACACTG TCATGCACGA CCGTCTCGCC AACATGCCGG GTTGCCACAC CTGGGAATCG 420

TTGGTCAATG CGGCGTACCG TTACTGCACC TGGGCTATGG AATGTATCTT CCTCGGCACC 480

GATGCAAATG TTCAATTCGG GATTGCTTGT TATCCCCACT TGAAAGAGCT CGAAGAGGCG 540

GCAAGGACGC TCAGGAAGGA TAGTATGGGC GAGTGGGATG ATGTGGTGCA GATTGGGGTG 600

GATAGGTTGT TGGAAATACT ACCTTGTGGT GAACGGACCT TGATCAACAA GAGAAGGATC 660

GAAAAGGAAC GACTGCAAGC GCTTCTAAAA GCTCAAACTC AAGTCAAGGC CAAGCATGAG 720

GCCAGATGTC AGGTGGCAAC CCCGATCCCA GCTCCAGCTG TCATTCCTGT CTCTGCACCT 780

GCTCCGGTTA TAATCAAGCG TAAAAAATAC AGTAGCCTAC GCAAGCTGAT GGGACGTGCG 840

GGGTCGGTGA TTGGCAGGCA GAAGATGCGG GCAAGGGCGT CTGCGATTAT CGAGAAGGGA 900

AAGGCCGGCT TCTACGATAG GCAAAAGGTC CAGCGCATGG AGCCGGCTCC ATCCATGTCT 960

CGTCCTGCGG GAGTGTCTTG TGTGTGATTG TCAGCTGGCC GTGAAGATAG TCTTGGAGCT 1020

GTGTATAGGG ATACTACATG ATGTGCATGC AGTGTGTTTT CAAAGCAATT GCCACGTGGT 1080

GTTCGCGTGG GTCGGAAACG AGGTTCGCGT TGTTATGGTT CTTGTTTTGA GATTCTCGCG 1140

CTGGCTACAC GTACGCACCA TACACTGCAG ACGACAGCGG CAAGCATACA GACGGCAACG 1200

CTGACATCCC AGCCACACAC ACC ATG TCC GCC ATC CGC GCA CTC ACG AGC 1250
Met Ser Ala Ile Arg Ala Leu Thr Ser
1               5

CCC GTC ACC GCC TGC TTC TCC ACC TCT CTC TCC CCG CAT GCC GCA TTC 1298
Pro Val Thr Ala Cys Phe Ser Thr Ser Leu Ser Pro His Ala Ala Phe

10                  15                  20                  25

CAC CGC TTC CTC CAT GCG CTC TCC GCC TCC ATC CGC CGC CTC GAC CTC        1346
His Arg Phe Leu His Ala Leu Ser Ala Ser Ile Arg Arg Leu Asp Leu
                30                  35                  40

TCC CGC GAC CCG CGC AAG ACC TTG GAC CGC CTC AGG CAG CAC AGG TTC        1394
Ser Arg Asp Pro Arg Lys Thr Leu Asp Arg Leu Arg Gln His Arg Phe
            45                  50                  55

ACC CTT GCC AAC ACT TTG CCC CGC GCG TTC ATG CTT CTG TGC GCC TCC        1442
Thr Leu Ala Asn Thr Leu Pro Arg Ala Phe Met Leu Leu Cys Ala Ser
        60                  65                  70

TAC AGT CTC TAC ATC ATG ACC ACC CCG CCC TTC CCA CTC AAG CTC GGG        1490
Tyr Ser Leu Tyr Ile Met Thr Thr Pro Pro Phe Pro Leu Lys Leu Gly
    75                  80                  85

ATA CCC ATC GCA TAC ATC GCC GCT GTC ATC TTC CCG ATC ACG TCG CAG        1538
Ile Pro Ile Ala Tyr Ile Ala Ala Val Ile Phe Pro Ile Thr Ser Gln
90                  95                  100                 105

TTT GTC TGG CCG GCG ACA CCC ATC TTT GCA TGG CTC ATC ACC TTT TTC        1586
Phe Val Trp Pro Ala Thr Pro Ile Phe Ala Trp Leu Ile Thr Phe Phe
                110                 115                 120

TCC GCC CGT TTC ATC CCT TCC GGC CGC CGG CCC GAA ATC CAC GTC GCC        1634
Ser Ala Arg Phe Ile Pro Ser Gly Arg Arg Pro Glu Ile His Val Ala
            125                 130                 135

CTT CTC CCA GCC CTT GAA TCC GTC CTC TAC GGT GCC AAC ATT TCT GAT        1682
Leu Leu Pro Ala Leu Glu Ser Val Leu Tyr Gly Ala Asn Ile Ser Asp
        140                 145                 150

CTC CAA ACA CGA TAT ACC AAC GCT TTC CTT GAT GTG GTC GCC TGG CTG        1730
Leu Gln Thr Arg Tyr Thr Asn Ala Phe Leu Asp Val Val Ala Trp Leu
    155                 160                 165

CCC TAT GGG GTA TTA CAT TTC ACC CTT CCA TTC GTC GTG GCT GTT ATC        1778
Pro Tyr Gly Val Leu His Phe Thr Leu Pro Phe Val Val Ala Val Ile
170                 175                 180                 185

CTT TGG TCA CTG GGA CCT AGG GGT GCG GTT CAG TTC TGG GGT TTG GCC        1826
Leu Trp Ser Leu Gly Pro Arg Gly Ala Val Gln Phe Trp Gly Leu Ala
                190                 195                 200

TTT GGG TGG ATG AAT TTG CTC GGT GTC GTC TGC CAG CTC CTC TTC CCT        1874
Phe Gly Trp Met Asn Leu Leu Gly Val Val Cys Gln Leu Leu Phe Pro
            205                 210                 215

GCT GCC GCG CCT TGG TAC GAA ATC ATT CAC GGT CTT ACC CCC GCC GAC        1922
Ala Ala Ala Pro Trp Tyr Glu Ile Ile His Gly Leu Thr Pro Ala Asp
        220                 225                 230

TAT TCC ATG GCT GGC TCT CCC GGC GGT CTC ATG CGT ATC GAC CGC GTC        1970
Tyr Ser Met Ala Gly Ser Pro Gly Gly Leu Met Arg Ile Asp Arg Val
    235                 240                 245

TTC CAT TCT TCA GGC TAT ACC AAC GCA TTC GGT TCC GCT CCT CTT GTT        2018
Phe His Ser Ser Gly Tyr Thr Asn Ala Phe Gly Ser Ala Pro Leu Val
250                 255                 260                 265

TTT GGC GCC TTC CCC TCC CTC CAT TCA GGA TGT GCC GTC ATG GAA GCC        2066
Phe Gly Ala Phe Pro Ser Leu His Ser Gly Cys Ala Val Met Glu Ala
                270                 275                 280

CTC TTC CTC TCC CAT TTC TTC CCA TCC CTC AAG GGC CTG TAT TGG GGC 2114
Leu Phe Leu Ser His Phe Phe Pro Ser Leu Lys Gly Leu Tyr Trp Gly
            285                 290                 295

TAT GTC GGT GTC CTC TGG TGG GCG ACC ATG TAC CTC TCC CAC CAC TAC 2162
Tyr Val Gly Val Leu Trp Trp Ala Thr Met Tyr Leu Ser His His Tyr
        300                 305                 310

CTC ATC GAC CTC GTT GGC GGA GCT TGT CTG AGC GTC TTG GTG TTC TAC 2210
Leu Ile Asp Leu Val Gly Gly Ala Cys Leu Ser Val Leu Val Phe Tyr
    315                 320                 325

TTG TGC ATG CCT GAA GGT TTC AAG GAC GTT GAT CAG ATA CAG TGG GAG 2258
Leu Cys Met Pro Glu Gly Phe Lys Asp Val Asp Gln Ile Gln Trp Glu
330                 335                 340                 345

GCG GTG GAA GGG GAT GGA TAC GAG ATG ATT GGC GGA CCC AGA ACA GGC 2306
Ala Val Glu Gly Asp Gly Tyr Glu Met Ile Gly Gly Pro Arg Thr Gly
                350                 355                 360

ACA GGT CCT GAG ATT GAT TTG GAT GAG GAA ATT AGA AAG TTG GAA GAA 2354
Thr Gly Pro Glu Ile Asp Leu Asp Glu Glu Ile Arg Lys Leu Glu Glu
            365                 370                 375

CAA GGC GAA GCG CTT TTT GAG CAG GTG ATA GGG GAT GAA GAG TCT CGG 2402
Gln Gly Glu Ala Leu Phe Glu Gln Val Ile Gly Asp Glu Glu Ser Arg
        380                 385                 390

ATC GAG ACA AGG GGA GAA GGA AAC AGT GGC GCG GGC GGG AAT CTG AGT 2450
Ile Glu Thr Arg Gly Glu Gly Asn Ser Gly Ala Gly Gly Asn Leu Ser
    395                 400                 405

GGG AAT GAA AGT GGT GAT AGT GCA GAT AGC GGT AGG GGA AAG GGC AAG 2498
Gly Asn Glu Ser Gly Asp Ser Ala Asp Ser Gly Arg Gly Lys Gly Lys
410                 415                 420                 425

GGG AAG GGG AAG GGG AAA CAA ACT GCC AAA AAG CCA AAG GCG AAG GAG 2546
Gly Lys Gly Lys Gly Lys Gln Thr Ala Lys Lys Pro Lys Ala Lys Glu
                430                 435                 440

CAA AGA TCG GTT AGC TGG GGA GAA ACA AAG GTG ATG GGT GAA GGA GCG 2594
Gln Arg Ser Val Ser Trp Gly Glu Thr Lys Val Met Gly Glu Gly Ala
            445                 450                 455

CAG GTG GCT TCA GAG AAT AGC TCT TAGATATCTG TTTTAAAACA GTAGTATACC 2648
Gln Val Ala Ser Glu Asn Ser Ser
        460                 465

CAACAATTTT TTGACGATAT ACCCTACCAC TATGATCTTA ATAATGCAGA ATAGAAAAAC 2708

ACCTTGCCCC TCTCGCAGGC GTCAACACAT GGAGTCTAGG CAGTTAAATC ACACTACATC 2768

ATCATCGCCG AAAAAAATAA ACCTAGTAAC TCCAACCCAA CCCTCCTGGT ACAGGCCTGT 2828

TCGTCCACCG ACTGATAATT CCCATCGCCC TCGCCCTCCT CACAAGTTTA CCCACCCGCC 2888

TTTGCGACTT CCAAGTCAAC CCGGTCTCCG CCCGACTTCT TATCTTGCCC ATCGGGTTGA 2948

CGAATTCATA GGCGAAATTA GGGTTCATAT CGTGCACCAA GGGGTTCGTT TTGGTGATGT 3008

AAAAAGGATC AAGCTTGACG GCGATTTTCT TGGGAGGACC AAGAAGAGGT GGACGGGGCA 3068

AAGGTCGAGC GGTGGGGTAG ATGGATTCTT GGGTGAATGC TTGAGGGGCA GTAAACTAGA 3128

AGAGTTGAGA AAACCCAAAG GAGTCAGCCT TTATGTCGAC 3168

(2) INFORMATION FOR SEQ ID NO:21:

(i) SEQUENCE CHARACTERISTICS:
(A) LENGTH: 465 amino acids
(B) TYPE: amino acid
(D) TOPOLOGY: linear

(ii) MOLECULE TYPE: protein

(xi) SEQUENCE DESCRIPTION: SEQ ID NO:21:

Met Ser Ala Ile Arg Ala Leu Thr Ser Pro Val Thr Ala Cys Phe Ser
1               5                   10                  15

Thr Ser Leu Ser Pro His Ala Ala Phe His Arg Phe Leu His Ala Leu
            20                  25                  30

Ser Ala Ser Ile Arg Arg Leu Asp Leu Ser Arg Asp Pro Arg Lys Thr
        35                  40                  45

Leu Asp Arg Leu Arg Gln His Arg Phe Thr Leu Ala Asn Thr Leu Pro
    50                  55                  60

Arg Ala Phe Met Leu Leu Cys Ala Ser Tyr Ser Leu Tyr Ile Met Thr
65                  70                  75                  80

Thr Pro Pro Phe Pro Leu Lys Leu Gly Ile Pro Ile Ala Tyr Ile Ala
                85                  90                  95

Ala Val Ile Phe Pro Ile Thr Ser Gln Phe Val Trp Pro Ala Thr Pro
            100                 105                 110

Ile Phe Ala Trp Leu Ile Thr Phe Phe Ser Ala Arg Phe Ile Pro Ser
        115                 120                 125

Gly Arg Arg Pro Glu Ile His Val Ala Leu Leu Pro Ala Leu Glu Ser
    130                 135                 140

Val Leu Tyr Gly Ala Asn Ile Ser Asp Leu Gln Thr Arg Tyr Thr Asn
145                 150                 155                 160

Ala Phe Leu Asp Val Val Ala Trp Leu Pro Tyr Gly Val Leu His Phe
                165                 170                 175

Thr Leu Pro Phe Val Val Ala Val Ile Leu Trp Ser Leu Gly Pro Arg
            180                 185                 190

Gly Ala Val Gln Phe Trp Gly Leu Ala Phe Gly Trp Met Asn Leu Leu
        195                 200                 205

Gly Val Val Cys Gln Leu Leu Phe Pro Ala Ala Ala Pro Trp Tyr Glu
    210                 215                 220

Ile Ile His Gly Leu Thr Pro Ala Asp Tyr Ser Met Ala Gly Ser Pro
225                 230                 235                 240

Gly Gly Leu Met Arg Ile Asp Arg Val Phe His Ser Ser Gly Tyr Thr
                245                 250                 255

Asn Ala Phe Gly Ser Ala Pro Leu Val Phe Gly Ala Phe Pro Ser Leu
            260                 265                 270

His Ser Gly Cys Ala Val Met Glu Ala Leu Phe Leu Ser His Phe Phe
        275                 280                 285

Pro Ser Leu Lys Gly Leu Tyr Trp Gly Tyr Val Gly Val Leu Trp Trp
    290                 295                 300

Ala Thr Met Tyr Leu Ser His His Tyr Leu Ile Asp Leu Val Gly Gly
305                 310                 315                 320

Ala Cys Leu Ser Val Leu Val Phe Tyr Leu Cys Met Pro Glu Gly Phe
                325                 330                 335

Lys Asp Val Asp Gln Ile Gln Trp Glu Ala Val Glu Gly Asp Gly Tyr
            340                 345                 350

Glu Met Ile Gly Gly Pro Arg Thr Gly Thr Gly Pro Glu Ile Asp Leu
        355                 360                 365

Asp Glu Glu Ile Arg Lys Leu Glu Glu Gln Gly Glu Ala Leu Phe Glu
    370                 375                 380

Gln Val Ile Gly Asp Glu Glu Ser Arg Ile Glu Thr Arg Gly Glu Gly
385                 390                 395                 400

Asn Ser Gly Ala Gly Gly Asn Leu Ser Gly Asn Glu Ser Gly Asp Ser
                405                 410                 415

Ala Asp Ser Gly Arg Gly Lys Gly Lys Gly Lys Gly Lys Gly Lys Gln
            420                 425                 430

Thr Ala Lys Lys Pro Lys Ala Lys Glu Gln Arg Ser Val Ser Trp Gly
        435                 440                 445

Glu Thr Lys Val Met Gly Glu Gly Ala Gln Val Ala Ser Glu Asn Ser
    450                 455                 460

Ser
465

(2) INFORMATION FOR SEQ ID NO:22:

     (i) SEQUENCE CHARACTERISTICS:
          (A) LENGTH: 3168 base pairs
          (B) TYPE: nucleic acid
          (C) STRANDEDNESS: single
          (D) TOPOLOGY: linear

    (ii) MOLECULE TYPE: mRNA

   (iii) HYPOTHETICAL: NO

    (iv) ANTI-SENSE: NO

    (xi) SEQUENCE DESCRIPTION: SEQ ID NO:22:

GUCGACAUAG UUACACUCCC CUCUACUCUU CAGACUACCC CUUCUGAUUA UCAGGAGACU      60

ACGACUACUC CGCCUACCUU GACCUUUGCA AACUCGGAGC CACGACCGAG UCCUUCCCUC     120

CAUCGUUCCC UGCGCAACAU CGGUUUGGCC GACGGCAGCA UGACUUGUGC CCCAGGCUAU     180

AUCCUCAUCU GCAUUCAUCA UGACCCCUUG UCUCGUUACA CGCUUGGCAU CAAUUUUACC     240

ACCUUUGUGG GUCUUUGCCC CACGACCCCU GCUAGUACCA GCACUUCCGG CGGUCCCGCC 300
UUUUCCUCUU UUACACCCAA GUUCGCCGAC CAUAUGCGAU GUCAUGUCAU CCAGCGCUUG 360
CUGCACACUG UCAUGCACGA CCGUCUCGCC AACAUGCCGG GUUGCCACAC CUGGGAAUCG 420
UUGGUCAAUG CGGCGUACCG UUACUGCACC UGGGCUAUGG AAUGUAUCUU CCUCGGCACC 480
GAUGCAAAUG UUCAAUUCGG GAUUGCUUGU UAUCCCCACU UGAAAGAGCU CGAAGAGGCG 540
GCAAGGACGC UCAGGAAGGA UAGUAUGGGC GAGUGGGAUG AUGUGGUGCA GAUUGGGGUG 600
GAUAGGUUGU UGGAAAUACU ACCUUGUGGU GAACGGACCU UGAUCAACAA GAGAAGGAUC 660
GAAAAGGAAC GACUGCAAGC GCUUCUAAAA GCUCAAACUC AAGUCAAGGC CAAGCAUGAG 720
GCCAGAUGUC AGGUGGCAAC CCCGAUCCCA GCUCCAGCUG UCAUUCCUGU CUCUGCACCU 780
GCUCCGGUUA UAAUCAAGCG UAAAAAAUAC AGUAGCCUAC GCAAGCUGAU GGGACGUGCG 840
GGGUCGGUGA UUGGCAGGCA GAAGAUGCGG GCAAGGGCGU CUGCGAUUAU CGAGAAGGGA 900
AAGGCCGGCU UCUACGAUAG GCAAAAGGUC CAGCGCAUGG AGCCGGCUCC AUCCAUGUCU 960
CGUCCUGCGG GAGUGUCUUG UGUGUGAUUG UCAGCUGGCC GUGAAGAUAG UCUUGGAGCU 1020
GUGUAUAGGG AUACUACAUG AUGUGCAUGC AGUGUGUUUU CAAAGCAAUU GCCACGUGGU 1080
GUUCGCGUGG GUCGGAAACG AGGUUCGCGU UGUUAUGGUU CUUGUUUUGA GAUUCUCGCG 1140
CUGGCUACAC GUACGCACCA UACACUGCAG ACGACAGCGG CAAGCAUACA GACGGCAACG 1200
CUGACAUCCC AGCCACACAC ACCAUGUCCG CCAUCCGCGC ACUCACGAGC CCCGUCACCG 1260
CCUGCUUCUC CACCUCUCUC UCCCCGCAUG CCGCAUUCCA CCGCUUCCUC CAUGCGCUCU 1320
CCGCCUCCAU CCGCCGCCUC GACCUCUCCC GCGACCCGCG CAAGACCUUG GACCGCCUCA 1380
GGCAGCACAG GUUCACCCUU GCCAACACUU UGCCCCGCGC GUUCAUGCUU CUGUGCGCCU 1440
CCUACAGUCU CUACAUCAUG ACCACCCCGC CCUUCCCACU CAAGCUCGGG AUACCCAUCG 1500
CAUACAUCGC CGCUGUCAUC UUCCCGAUCA CGUCGCAGUU UGUCUGGCCG GCGACACCCA 1560
UCUUUGCAUG GCUCAUCACC UUUUUCUCCG CCCGUUUCAU CCCUUCCGGC CGCCGGCCCG 1620
AAAUCCACGU CGCCCUUCUC CCAGCCCUUG AAUCCGUCCU CUACGGUGCC AACAUUUCUG 1680
AUCUCCAAAC ACGAUAUACC AACGCUUUCC UUGAUGUGGU CGCCUGGCUG CCCUAUGGGG 1740
UAUUACAUUU CACCCUUCCA UUCGUCGUGG CUGUUAUCCU UUGGUCACUG GGACCUAGGG 1800
GUGCGGUUCA GUUCUGGGGU UUGGCCUUUG GGUGGAUGAA UUUGCUCGGU GUCGUCUGCC 1860
AGCUCCUCUU CCCUGCUGCC GCGCCUUGGU ACGAAAUCAU UCACGGUCUU ACCCCCGCCG 1920
ACUAUUCCAU GGCUGGCUCU CCCGGCGGUC UCAUGCGUAU CGACCGCGUC UUCCAUUCUU 1980
CAGGCUAUAC CAACGCAUUC GGUUCCGCUC CUCUUGUUUU UGGCGCCUUC CCCUCCCUCC 2040
AUUCAGGAUG UGCCGUCAUG GAAGCCCUCU UCCUCUCCCA UUUCUUCCCA UCCCUCAAGG 2100
GCCUGUAUUG GGGCUAUGUC GGUGUCCUCU GGUGGGCGAC CAUGUACCUC UCCCACCACU 2160

ACCUCAUCGA CCUCGUUGGC GGAGCUUGUC UGAGCGUCUU GGUGUUCUAC UUGUGCAUGC 2220

CUGAAGGUUU CAAGGACGUU GAUCAGAUAC AGUGGGAGGC GGUGGAAGGG GAUGGAUACG 2280

AGAUGAUUGG CGGACCCAGA ACAGGCACAG GUCCUGAGAU UGAUUUGGAU GAGGAAAUUA 2340

GAAAGUUGGA AGAACAAGGC GAAGCGCUUU UUGAGCAGGU GAUAGGGGAU GAAGAGUCUC 2400

GGAUCGAGAC AAGGGGAGAA GGAAACAGUG GCGCGGGCGG GAAUCUGAGU GGGAAUGAAA 2460

GUGGUGAUAG UGCAGAUAGC GGUAGGGGAA AGGGCAAGGG GAAGGGGAAG GGGAAACAAA 2520

CUGCCAAAAA GCCAAAGGCG AAGGAGCAAA GAUCGGUUAG CUGGGGAGAA ACAAAGGUGA 2580

UGGGUGAAGG AGCGCAGGUG GCUUCAGAGA AUAGCUCUUA GAUAUCUGUU UUAAAACAGU 2640

AGUAUACCCA ACAAUUUUUU GACGAUAUAC CCUACCACUA UGAUCUUAAU AAUGCAGAAU 2700

AGAAAAACAC CUUGCCCCUC UCGCAGGCGU CAACACAUGG AGUCUAGGCA GUUAAAUCAC 2760

ACUACAUCAU CAUCGCCGAA AAAAAUAAAC CUAGUAACUC CAACCCAACC CUCCUGGUAC 2820

AGGCCUGUUC GUCCACCGAC UGAUAAUUCC CAUCGCCCUC GCCCUCCUCA CAAGUUUACC 2880

CACCCGCCUU UGCGACUUCC AAGUCAACCC GGUCUCCGCC CGACUUCUUA UCUUGCCCAU 2940

CGGGUUGACG AAUUCAUAGG CGAAAUUAGG GUUCAUAUCG UGCACCAAGG GGUUCGUUUU 3000

GGUGAUGUAA AAAGGAUCAA GCUUGACGGC GAUUUUCUUG GGAGGACCAA GAAGAGGUGG 3060

ACGGGGCAAA GGUCGAGCGG UGGGGUAGAU GGAUUCUUGG GUGAAUGCUU GAGGGGCAGU 3120

AAACUAGAAG AGUUGAGAAA ACCCAAAGGA GUCAGCCUUU AUGUCGAC 3168